# FASCICULE DE BREVET EUROPEEN

(11) **EP 1 200 597 B1**
(45) Date de publication et mention de la délivrance du brevet: **14.06.2006**
(21) Numéro de dépôt: 00949686.0
(22) Date de dépôt: 07.07.2000
(51) Int. Cl.: C12N 15/53, C12N 15/60, C12P 7/18, C12N 9/88, C12N 9/04, C12N 1/19, C12N 1/21, C07K 16/40, C12R 1/145

(54) **PROCEDE DE PREPARATION DU 1,3-PROPANEDIOL PAR UN MICRO-ORGANISME RECOMBINANT EN L'ABSENCE DE COENZYME B12 OU DE L'UN DE SES PRECURSEURS**
VERFAHREN ZUR HERSTELLUNG VON 1,3-PROPANEDIOL DURCH EINEN REKOMBINANTEN MICRO-ORGANISMUS IN ABWESENHEIT VON COENZYM B12
METHOD FOR PREPARING 1,3-PROPANEDIOL BY A RECOMBINANT MICRO-ORGANISM IN THE ABSENCE OF COENZYME B12 OR ONE OF ITS PRECURSORS

(30) Priorité: 09.07.1999 FR 9908939
(43) Date de publication de la demande: 02.05.2002
(73) Titulaire: INSTITUT NATIONAL DE LA RECHERCHE AGRONOMIQUE (INRA), 75341 Paris Cédéx 07 (FR); Institut National des Sciences Appliquees de Toulouse, 31077 Toulouse Cedex 4 (FR); CENTRE NATIONAL DE LA RECHERCHE SCIENTIFIQUE, 75794 Paris Cedex 16 (FR)
(72) Inventeur: Sarcabal, Patricia, 31400 Toulouse (FR); Croux, Christian, 31320 Castanet Tolosan (FR); Soucaille, Philippe, 31450 Deyme (FR)
(74) Mandataire: Catherine, Alain
(86) Numéro de dépôt international: PCT/FR2000/001981
(87) Numéro de publication internationale: WO 2001/004324

(56) Documents cités:
- LUERS F ET AL: "Glycerol conversion to 1,3-propanediol by Clostridium pasteurianum: cloning and expression of the gene encoding 1,3-propanediol dehydrogenase." FEMS MICROBIOLOGY LETTERS, vol. 154, 15 septembre 1997 (1997-09-15), pages 337-345, XP000901192 AMSTERDAM, NL ISSN: 0378-1097
- MACIS L ET AL: "Properties and sequence of the coenzyme B12-dependent glycerol dehydratase of Clostridium pasteurianum" FEMS MICROBIOLOGY LETTERS, vol. 164, 1 juillet 1998 (1998-07-01), pages 21-28, XP000901179 AMSTERDAM, NL ISSN: 0378-1097
- REIMANN A ET AL: "1,3-propanediol formation with product-tolerant mutants of Clostridium butyricum DSM 5431 in continuous culture: productivity, carbon and electron flow." JOURNAL OF APPLIED MICROBIOLOGY., vol. 84, no. 6, juin 1998 (1998-06), pages 1125-1130, XP000901178 OXFORD, GB ISSN: 1364-5072
- WEIDNER G ET AL: "MOLECULAR CHARACTERIZATION OF THE GENES ENCODING PYRUVATE FORMATE-LYASE AND ITS ACTIVATING ENZYME OF CLOSTRIDIUM PASTEURIANUM" JOURNAL OF BACTERIOLOGY,US,WASHINGTON, DC, vol. 178, no. 8, 1 avril 1996 (1996-04-01), pages 2440-2444, XP002055590 ISSN: 0021-9193
- SKRALY F A ET AL: "Construction and characterization of a 1,3-propanediol operon" APPLIED AND ENVIRONMENTAL MICROBIOLOGY, vol. 64, no. 1, janvier 1998 (1998-01), pages 98-105, XP002134649 WASHINGTON,DC, US ISSN: 0099-2240

### LISTE DE SEQUENCES

<110> INSTITUT NATIONAL DE LA RECHERCHE AGRONOMIQUE (INRA)
<120> Procédé de préparation du 1,3-propanediol à partir d'un microorganisme recombinant , en l'absence de coenzyme B12 ou de l'un de ses précurseurs.
<130> 1,3-propanediol INRA
<140>
   <141>
<160> 10
<170> PatentIn Ver. 2.1
<210> 1
   <211> 2364
   <212> ADN
   <213> Clostridium butyricum
<400> 1
   atgataagta aaggatttag tacccaaaca gaaagaataa atattttaaa ggctcaaata 60
   ttaaatgcta aaccatgtgt tgaatcagaa agagcaatat taataacaga atcatttaaa 120
   caaacagaag gccagccagc aattttaaga agagcattgg cattgaaaca catacttgaa 180
   aatatcccta taacaattag agatcaagaa cttatagtgg gaagtttaac taaagaacca 240
   aggtcttcac aagtatttcc tgagttttct aataagtggt tacaagatga attggataga 300
   ttaaataaga gaactggaga tgcattccaa atttcagaag aaagtaaaga aaaattaaaa 360
   gatgtctttg agtattggaa tggaaagaca acaagtgagt tagcaacttc atatatgaca 420
   gaggaaacaa gagaggcagt aaattgtgaa gtatttactg taggaaacta ctattataat 480
   ggcgtaggac atgtatctgt agattatgga aaagtattaa gggttggatt taatgggatt 540
   ataaatgagg ctaaggaaca attagaaaaa aacaggagta tagatcctga ttttataaag 600
   aaagaaaaat tcctaaatag tgttattatc tcatgcgaag ctgcaataac atatgtaaat 660
   agatatgcta aaaaggctaa agagattgca gataatacaa gtgatgcaaa aagaaaagct 720
   gaattaaatg aaatagcaaa aatttgttca aaagtttcag gagagggagc taaatctttc 780
   tatgaagcat gtcaattatt ttggtttatt catgcaataa taaatataga atctaatgga 840
   cattctattt ctccagctag atttgatcaa tacatgtatc catattatga aaatgataaa 900
   aatataacag ataagtttgc tcaagaatta atagattgta tctggattaa attaaatgat 960
   attaataaag taagagatga gatttcaact aaacattttg gtggttaccc aatgtatcaa 1020
   aaattaattg ttgggggtca aaattcagaa ggaaaagatg caactaataa agtatcatat 1080
   atggcattag aagcagctgt ccatgtaaag ttgcctcagc catctttgtc agtaagaata 1140
   tggaataaga ctccagatga atttttgctt agagcagcag aattaactag agaagggtta 1200
   ggacttcctg cttattataa tgatgaagtt attattccag cattagtttc tagaggtctt 1260
   acattagaag atgcaagaga ctacggaata attggatgtg ttgaaccaca aaagccagga 1320
   aaaacagaag gatggcatga ttcagcattc tttaatcttg caagaatagt agagttaact 1380
   ataaattctg gatttgataa aaataaacag attggaccta aaactcaaaa ttttgaagaa 1440
   atgaaatcct ttgatgaatt catgaaagct tataaagctc aaatggagta ttttgtaaaa 1500
   catatgtgct gtgctgataa ttgcatagat attgcacatg cagaaagagc tccattacct 1560
   ttcttgtcat caatggttga taattgtatc ggaaaaggaa agagccttca agatggtggt 1620
   gcagaatata acttcagtgg accacaaggt gttggagtag ctaatattgg agattcatta 1680
   gttgcagtta aaaaaattgt gtttgatgaa aataagatta ctccttcaga attaaagaaa 1740
   acattaaata atgattttaa aaattcagaa gaaatacaag ccttactaaa aaatgctcct 1800
   aagtttggaa atgatattga tgaagttgat aatttagcta gagagggtgc attagtatac 1860
   tgtagagaag ttaataaata tacaaatcca aggggaggaa attttcaacc aggattatat 1920
   ccatcttcaa ttaatgtata ttttggaagc ttaacaggtg ctactccaga tggaaggaaa 1980
   tccggacaac cattagctga tggggtttct ccatcaagag gctgtgatgt atctggacct 2040
   actgcagctt gtaactcagt tagtaaatta gatcatttta tagcttcaaa tggaacttta 2100
   tttaatcaaa aattccatcc gtcagcatta aaaggtgata atggattaat gaatttatca 2160
   tcattaataa gaagttattt tgatcaaaag ggatttcatg ttcaatttaa tgtaatagat 2220
   aaaaaaatat tacttgcagc acaaaaaaat cctgaaaaat atcaagattt aattgttaga 2280
   gttgcaggat atagtgcaca gttcatttct ttagataaat ctattcaaaa tgatattatt 2340
   gcaagaactg aacatgttat gtaa                                        2364
<210> 2
   <211> 915
   <212> ADN
   <213> Clostridium butyricum
<400> 2
   atgagtaagg agataaaagg cgttttattt aacatacaaa aattttcgtt acatgatggg 60
   cctggaataa gaactatagt attttttaag ggatgttcaa tgtcgtgctt atggtgcagt 120
   aatccagaat cccaagatat taaacctcaa gtaatgttta ataaaaattt atgtacaaaa 180
   tgtggaagat gtaaatctca atgtaaaagt gcaggtattg atatgaattc agaatatagg 240
   atagataaaa gcaaatgtac agagtgtaca aaatgtgttg ataattgctt aagcggggca 300
   cttgttattg aaggaaggaa ttacagtgtt gaagacgtta taaaggaatt gaaaaaagat 360
   agtgttcaat atagaagatc aaacggtgga attacactat ctggagggga agtattactt 420
   caaccagatt ttgcagtgga gcttttaaaa gagtgtaaat catatggctg gcacactgcc 480
   attgaaacag caatgtatgt taatagtgaa tctgtaaaaa aagtaattcc atatatagat 540
   ctggctatga ttgatataaa aagtatgaat gatgaaatcc ataggaaatt tacaggagtg 600
   agtaacgaaa taatattaca aaacattaaa ttaagtgatg aattagctaa agaaataata 660
   atcagaattc ctgtaataga aggatttaat gcagatttac aaagtatagg agcaatagct 720
   caattttcaa aatcattaac aaatcttaaa agaatagatc ttcttccata ccataattat 780
   ggagaaaata agtatcaagc aattggaaga gagtattctt tgaaagaact aaaatcacct 840
   agtaaagaca aaatggaaag attaaaagct ttagttgaaa tcatgggaat accgtgcaca 900
   attggagctg agtaa                                                  915
<210> 3
   <211> 28
   <212> ADN
   <213> Clostridium butyricum
<400> 3
   tagataaaac aaacaaaaat gttattat 28
<210> 4
   <211> 1158
   <212> ADN
   <213> Clostridium butyricum
<400> 4
   atgagaatgt atgattattt agtaccaagt gtaaacttta tgggagcaaa ttcagtatca 60
   gtagtaggtg aaagatgcaa aatattaggt ggaaaaaaag cattgatagt tacagataag 120
   tttctaaaag atatggaagg tggagctgtt gaattaacag ttaaatattt aaaagaagct 180
   ggattagatg ttgtatatta tgacggagtt gaaccaaatc caaaagatgt taatgttata 240
   gaaggattaa aaatatttaa agaagaaaat tgtgacatga tagtaactgt aggtggagga 300
   agttcgcatg attgcggtaa gggaatagga attgctgcaa cacatgaagg agatctttat 360
   gattatgcag gaatagaaac acttgtcaat ccattgccac caatagtagc tgtaaatact 420
   actgcaggaa ctgctagtga attaactcgt cattgtgtat tgactaatac aaaaaagaaa 480
   ataaaatttg ttatagttag ctggagaaat ttgcctctag tatctataaa tgatccaatg 540
   cttatggtca aaaaacctgc aggattaaca gcagctacag gaatggatgc tttaacacat 600
   gcaatagaag catatgtatc aaaagatgca aatccagtaa cagatgcttc agcaatacaa 660
   gctattaaat taatttcaca aaatttaaga caagctgtag ctttaggaga aaatcttgaa 720
   gcaagagaaa atatggctta tgcatcatta ctagcaggaa tggcatttaa taatgctaat 780
   ttaggatatg tacatgcaat ggctcatcaa ttagggggac tgtatgatat ggcacatggt 840
   gttgctaatg caatgctatt accacatgtt gaacgttata atatgctatc aaatcctaag 900
   aagtttgcag atatagcaga atttatggga gaaaatatat ctggactttc tgtaatggaa 960
   gcagcagaga aagccataaa tgcaatgttc aggctttcag aggatgttgg aattccgaaa 1020
   agtctaaagg agatgggagt gaaacaagaa gattttgagc atatggcaga actagctctt 1080
   ttagatggaa atgcctttag caatccaaga aaaggaaatg caaaagatat tataaatatt 1140
   tttaaggctg cttattaa                                              1158
<210> 5
   <211> 4963
   <212> ADN
   <213> Clostridium butyricum
<400> 5
   gaataaaagt tatctaataa tgataaaagt cattattaga taacttttta ttttaaaata 60
   actactaata aaaagttcaa agaatattac agtagacatt tgaaagaatg caatgataaa 120
   caattgtatt agttttaact ttagataaaa caaacaaaaa tgttattatt agccaagaaa 180
   atactgttac ataaaaaacg agaaaaacat agcaaaagag taccaatatt aagcaataaa 240
   gtttgttaaa atattatcaa taaaatgata agattagata aaccaagtaa gaatgtgatt 300
   ggaggagtaa aaatgataag taaaggattt agtacccaaa cagaaagaat aaatatttta 360
   aaggctcaaa tattaaatgc taaaccatgt gttgaatcag aaagagcaat attaataaca 420
   gaatcattta aacaaacaga aggccagcca gcaattttaa gaagagcatt ggcattgaaa 480
   cacatacttg aaaatatccc tataacaatt agagatcaag aacttatagt gggaagttta 540
   actaaagaac caaggtcttc acaagtattt cctgagtttt ctaataagtg gttacaagat 600
   gaattggata gattaaataa gagaactgga gatgcattcc aaatttcaga agaaagtaaa 660
   gaaaaattaa aagatgtctt tgagtattgg aatggaaaga caacaagtga gttagcaact 720
   tcatatatga cagaggaaac aagagaggca gtaaattgtg aagtatttac tgtaggaaac 780
   tactattata atggcgtagg acatgtatct gtagattatg gaaaagtatt aagggttgga 840
   tttaatggga ttataaatga ggctaaggaa caattagaaa aaaacaggag tatagatcct 900
   gattttataa agaaagaaaa attcctaaat agtgttatta tctcatgcga agctgcaata 960
   acatatgtaa atagatatgc taaaaaggct aaagagattg cagataatac aagtgatgca 1020
   aaaagaaaag ctgaattaaa tgaaatagca aaaatttgtt caaaagtttc aggagaggga 1080
   gctaaatctt tctatgaagc atgtcaatta ttttggttta ttcatgcaat aataaatata 1140
   gaatctaatg gacattctat ttctccagct agatttgatc aatacatgta tccatattat 1200
   gaaaatgata aaaatataac agataagttt gctcaagaat taatagattg tatctggatt 1260
   aaattaaatg atattaataa agtaagagat gagatttcaa ctaaacattt tggtggttac 1320
   ccaatgtatc aaaaattaat tgttgggggt caaaattcag aaggaaaaga tgcaactaat 1380
   aaagtatcat atatggcatt agaagcagct gtccatgtaa agttgcctca gccatctttg 1440
   tcagtaagaa tatggaataa gactccagat gaatttttgc ttagagcagc agaattaact 1500
   agagaagggt taggacttcc tgcttattat aatgatgaag ttattattcc agcattagtt 1560
   tctagaggtc ttacattaga agatgcaaga gactacggaa taattggatg tgttgaacca 1620
   caaaagccag gaaaaacaga aggatggcat gattcagcat tctttaatct tgcaagaata 1680
   gtagagttaa ctataaattc tggatttgat aaaaataaac agattggacc taaaactcaa 1740
   aattttgaag aaatgaaatc ctttgatgaa ttcatgaaag cttataaagc tcaaatggag 1800
   tattttgtaa aacatatgtg ctgtgctgat aattgcatag atattgcaca tgcagaaaga 1860
   gctccattac ctttcttgtc atcaatggtt gataattgta tcggaaaagg aaagagcctt 1920
   caagatggtg gtgcagaata taacttcagt ggaccacaag gtgttggagt agctaatatt 1980
   ggagattcat tagttgcagt taaaaaaatt gtgtttgatg aaaataagat tactccttca 2040
   gaattaaaga aaacattaaa taatgatttt aaaaattcag aagaaataca agccttacta 2100
   aaaaatgctc ctaagtttgg aaatgatatt gatgaagttg ataatttagc tagagagggt 2160
   gcattagtat actgtagaga agttaataaa tatacaaatc caaggggagg aaattttcaa 2220
   ccaggattat atccatcttc aattaatgta tattttggaa gcttaacagg tgctactcca 2280
   gatggaagga aatccggaca accattagct gatggggttt ctccatcaag aggctgtgat 2340
   gtatctggac ctactgcagc ttgtaactca gttagtaaat tagatcattt tatagcttca 2400
   aatggaactt tatttaatca aaaattccat ccgtcagcat taaaaggtga taatggatta 2460
   atgaatttat catcattaat aagaagttat tttgatcaaa agggatttca tgttcaattt 2520
   aatgtaatag ataaaaaaat attacttgca gcacaaaaaa atcctgaaaa atatcaagat 2580
   ttaattgtta gagttgcagg atatagtgca cagttcattt ctttagataa atctattcaa 2640
   aatgatatta ttgcaagaac tgaacatgtt atgtaaagac agcttttaaa ggggataaaa 2700
   gtaatgagta aggagataaa aggcgtttta tttaacatac aaaaattttc gttacatgat 2760
   gggcctggaa taagaactat agtatttttt aagggatgtt caatgtcgtg cttatggtgc 2820
   agtaatccag aatcccaaga tattaaacct caagtaatgt ttaataaaaa tttatgtaca 2880
   aaatgtggaa gatgtaaatc tcaatgtaaa agtgcaggta ttgatatgaa ttcagaatat 2940
   aggatagata aaagcaaatg tacagagtgt acaaaatgtg ttgataattg cttaagcggg 3000
   gcacttgtta ttgaaggaag gaattacagt gttgaagacg ttataaagga attgaaaaaa 3060
   gatagtgttc aatatagaag atcaaacggt ggaattacac tatctggagg ggaagtatta 3120
   cttcaaccag attttgcagt ggagctttta aaagagtgta aatcatatgg ctggcacact 3180
   gccattgaaa cagcaatgta tgttaatagt gaatctgtaa aaaaagtaat tccatatata 3240
   gatctggcta tgattgatat aaaaagtatg aatgatgaaa tccataggaa atttacagga 3300
   gtgagtaacg aaataatatt acaaaacatt aaattaagtg atgaattagc taaagaaata 3360
   ataatcagaa ttcctgtaat agaaggattt aatgcagatt tacaaagtat aggagcaata 3420
   gctcaatttt caaaatcatt aacaaatctt aaaagaatag atcttcttcc ataccataat 3480
   tatggagaaa ataagtatca agcaattgga agagagtatt ctttgaaaga actaaaatca 3540
   cctagtaaag acaaaatgga aagattaaaa gctttagttg aaatcatggg aataccgtgc 3600
   acaattggag ctgagtaata gtagctttac atcagatatt ttaaaaacaa ttttaaatta 3660
   aaaggagaag attgcatatg agaatgtatg attatttagt accaagtgta aactttatgg 3720
   gagcaaattc agtatcagta gtaggtgaaa gatgcaaaat attaggtgga aaaaaagcat 3780
   tgatagttac agataagttt ctaaaagata tggaaggtgg agctgttgaa ttaacagtta 3840
   aatatttaaa agaagctgga ttagatgttg tatattatga cggagttgaa ccaaatccaa 3900
   aagatgttaa tgttatagaa ggattaaaaa tatttaaaga agaaaattgt gacatgatag 3960
   taactgtagg tggaggaagt tcgcatgatt gcggtaaggg aataggaatt gctgcaacac 4020
   atgaaggaga tctttatgat tatgcaggaa tagaaacact tgtcaatcca ttgccaccaa 4080
   tagtagctgt aaatactact gcaggaactg ctagtgaatt aactcgtcat tgtgtattga 4140
   ctaatacaaa aaagaaaata aaatttgtta tagttagctg gagaaatttg cctctagtat 4200
   ctataaatga tccaatgctt atggtcaaaa aacctgcagg attaacagca gctacaggaa 4260
   tggatgcttt aacacatgca atagaagcat atgtatcaaa agatgcaaat ccagtaacag 4320
   atgcttcagc aatacaagct attaaattaa tttcacaaaa tttaagacaa gctgtagctt 4380
   taggagaaaa tcttgaagca agagaaaata tggcttatgc atcattacta gcaggaatgg 4440
   catttaataa tgctaattta ggatatgtac atgcaatggc tcatcaatta gggggactgt 4500
   atgatatggc acatggtgtt gctaatgcaa tgctattacc acatgttgaa cgttataata 4560
   tgctatcaaa tcctaagaag tttgcagata tagcagaatt tatgggagaa aatatatctg 4620
   gactttctgt aatggaagca gcagagaaag ccataaatgc aatgttcagg ctttcagagg 4680
   atgttggaat tccgaaaagt ctaaaggaga tgggagtgaa acaagaagat tttgagcata 4740
   tggcagaact agctctttta gatggaaatg cctttagcaa tccaagaaaa ggaaatgcaa 4800
   aagatattat aaatattttt aaggctgctt attaattaat actatttaaa ggattcaaag 4860
   taaaagataa aagatatata tattagattt aagattttat tataggctaa caacaaagaa 4920
   caagttaagt attaaactta gcttgttctt tgttgtttat ttt                   4963
<210> 6
   <211> 787
   <212> PRT
   <213> Clostridium butyricum
<400> 6
   Met Ile Ser Lys Gly Phe Ser Thr Gln Thr Glu Arg Ile Asn Ile Leu
   1               5                   10                  15
   Lys Ala Gln Ile Leu Asn Ala Lys Pro Cys Val Glu Ser Glu Arg Ala
               20                  25                  30
   Ile Leu Ile Thr Glu Ser Phe Lys Gln Thr Glu Gly Gln Pro Ala Ile
           35                  40                  45
   Leu Arg Arg Ala Leu Ala Leu Lys His Ile Leu Glu Asn Ile Pro Ile
       50                  55                  60
   Thr Ile Arg Asp Gln Glu Leu Ile Val Gly Ser Leu Thr Lys Glu Pro
   65                  70                  75                  80
   Arg Ser Ser Gln Val Phe Pro Glu Phe Ser Asn Lys Trp Leu Gln Asp
                   85                  90                  95
   Glu Leu Asp Arg Leu Asn Lys Arg Thr Gly Asp Ala Phe Gln Ile Ser
               100                 105                 110
   Glu Glu Ser Lys Glu Lys Leu Lys Asp Val Phe Glu Tyr Trp Asn Gly
   115 120 125
   Lys Thr Thr Ser Glu Leu Ala Thr Ser Tyr Met Thr Glu Glu Thr Arg
   130 135 140
   Glu Ala Val Asn Cys Glu Val Phe Thr Val Gly Asn Tyr Tyr Tyr Asn
   145 150 155 160
   Gly Val Gly His Val Ser Val Asp Tyr Gly Lys Val Leu Arg Val Gly
   165 170 175
   Phe Asn Gly Ile Ile Asn Glu Ala Lys Glu Gln Leu Glu Lys Asn Arg
   180 185 190
   Ser Ile Asp Pro Asp Phe Ile Lys Lys Glu Lys Phe Leu Asn Ser Val
   195 200 205
   Ile Ile Ser Cys Glu Ala Ala Ile Thr Tyr Val Asn Arg Tyr Ala Lys
   210 215 220
   Lys Ala Lys Glu Ile Ala Asp Asn Thr Ser Asp Ala Lys Arg Lys Ala
   225 230 235 240
   Glu Leu Asn Glu Ile Ala Lys Ile Cys Ser Lys Val Ser Gly Glu Gly
   245 250 255
   Ala Lys Ser Phe Tyr Glu Ala Cys Gln Leu Phe Trp Phe Ile His Ala
   260 265 270
   Ile Ile Asn Ile Glu Ser Asn Gly His Ser Ile Ser Pro Ala Arg Phe
   275 280 285
   Asp Gln Tyr Met Tyr Pro Tyr Tyr Glu Asn Asp Lys Asn Ile Thr Asp
   290 295 300
   Lys Phe Ala Gln Glu Leu Ile Asp Cys Ile Trp Ile Lys Leu Asn Asp
   305 310 315 320
   Ile Asn Lys Val Arg Asp Glu Ile Ser Thr Lys His Phe Gly Gly Tyr
   325 330 335
   Pro Met Tyr Gln Lys Leu Ile Val Gly Gly Gln Asn Ser Glu Gly Lys
   340 345 350
   Asp Ala Thr Asn Lys Val Ser Tyr Met Ala Leu Glu Ala Ala Val His
   355 360 365
   Val Lys Leu Pro Gln Pro Ser Leu Ser Val Arg Ile Trp Asn Lys Thr
   370 375 380
   Pro Asp Glu Phe Leu Leu Arg Ala Ala Glu Leu Thr Arg Glu Gly Leu
   385 390 395 400
   Gly Leu Pro Ala Tyr Tyr Asn Asp Glu Val Ile Ile Pro Ala Leu Val
   405 410 415
   Ser Arg Gly Leu Thr Leu Glu Asp Ala Arg Asp Tyr Gly Ile Ile Gly
   420 425 430
   Cys Val Glu Pro Gln Lys Pro Gly Lys Thr Glu Gly Trp His Asp Ser
   435 440 445
   Ala Phe Phe Asn Leu Ala Arg Ile Val Glu Leu Thr Ile Asn Ser Gly
   450 455 460
   Phe Asp Lys Asn Lys Gln Ile Gly Pro Lys Thr Gln Asn Phe Glu Glu
   465 470 475 480
   Met Lys Ser Phe Asp Glu Phe Met Lys Ala Tyr Lys Ala Gln Met Glu
   485 490 495
   Tyr Phe Val Lys His Met Cys Cys Ala Asp Asn Cys Ile Asp Ile Ala
   500 505 510
   His Ala Glu Arg Ala Pro Leu Pro Phe Leu Ser Ser Met Val Asp Asn
   515 520 525
   Cys Ile Gly Lys Gly Lys Ser Leu Gln Asp Gly Gly Ala Glu Tyr Asn
   530 535 540
   Phe Ser Gly Pro Gln Gly Val Gly Val Ala Asn Ile Gly Asp Ser Leu
   545 550 555 560
   Val Ala Val Lys Lys Ile Val Phe Asp Glu Asn Lys Ile Thr Pro Ser
   565 570 575
   Glu Leu Lys Lys Thr Leu Asn Asn Asp Phe Lys Asn Ser Glu Glu Ile
   580 585 590
   Gln Ala Leu Leu Lys Asn Ala Pro Lys Phe Gly Asn Asp Ile Asp Glu
   595 600 605
   Val Asp Asn Leu Ala Arg Glu Gly Ala Leu Val Tyr Cys Arg Glu Val
   610 615 620
   Asn Lys Tyr Thr Asn Pro Arg Gly Gly Asn Phe Gln Pro Gly Leu Tyr
   625 630 635 640
   Pro Ser Ser Ile Asn Val Tyr Phe Gly Ser Leu Thr Gly Ala Thr Pro
   645 650 655
   Asp Gly Arg Lys Ser Gly Gln Pro Leu Ala Asp Gly Val Ser Pro Ser
   660 665 670
   Arg Gly Cys Asp Val Ser Gly Pro Thr Ala Ala Cys Asn Ser Val Ser
   675 680 685
   Lys Leu Asp His Phe Ile Ala Ser Asn Gly Thr Leu Phe Asn Gln Lys
   690 695 700
   Phe His Pro Ser Ala Leu Lys Gly Asp Asn Gly Leu Met Asn Leu Ser
   705 710 715 720
   Ser Leu Ile Arg Ser Tyr Phe Asp Gln Lys Gly Phe His Val Gln Phe
   725 730 735
   Asn Val Ile Asp Lys Lys Ile Leu Leu Ala Ala Gln Lys Asn Pro Glu
   740 745 750
   Lys Tyr Gln Asp Leu Ile Val Arg Val Ala Gly Tyr Ser Ala Gln Phe
   755 760 765
   Ile Ser Leu Asp Lys Ser Ile Gln Asn Asp Ile Ile Ala Arg Thr Glu
   770 775 780
   His Val Met
   785
<210> 7
   <211> 304
   <212> PRT
   <213> Clostridium butyricum
<400> 7
   Met Ser Lys Glu Ile Lys Gly Val Leu Phe Asn Ile Gln Lys Phe Ser
    1               5                  10                  15
   Leu His Asp Gly Pro Gly Ile Arg Thr Ile Val Phe Phe Lys Gly Cys
               20                  25                  30
   Ser Met Ser Cys Leu Trp Cys Ser Asn Pro Glu Ser Gln Asp Ile Lys
           35                  40                  45
   Pro Gln Val Met Phe Asn Lys Asn Leu Cys Thr Lys Cys Gly Arg Cys
       50                  55                  60
   Lys Ser Gln Cys Lys Ser Ala Gly Ile Asp Met Asn Ser Glu Tyr Arg
   65                  70                  75                  80
   Ile Asp Lys Ser Lys Cys Thr Glu Cys Thr Lys Cys Val Asp Asn Cys
                   85                  90                  95
   Leu Ser Gly Ala Leu Val Ile Glu Gly Arg Asn Tyr Ser Val Glu Asp
               100                 105                 110
   Val Ile Lys Glu Leu Lys Lys Asp Ser Val Gln Tyr Arg Arg Ser Asn
           115                 120                 125
   Gly Gly Ile Thr Leu Ser Gly Gly Glu Val Leu Leu Gln Pro Asp Phe
       130                 135                 140
   Ala Val Glu Leu Leu Lys Glu Cys Lys Ser Tyr Gly Trp His Thr Ala
   145 150 155 160
   Ile Glu Thr Ala Met Tyr Val Asn Ser Glu Ser Val Lys Lys Val Ile
   165 170 175
   Pro Tyr Ile Asp Leu Ala Met Ile Asp Ile Lys Ser Met Asn Asp Glu
   180 185 190
   Ile His Arg Lys Phe Thr Gly Val Ser Asn Glu Ile Ile Leu Gln Asn
   195 200 205
   Ile Lys Leu Ser Asp Glu Leu Ala Lys Glu Ile Ile Ile Arg Ile Pro
   210 215 220
   Val Ile Glu Gly Phe Asn Ala Asp Leu Gln Ser Ile Gly Ala Ile Ala
   225 230 235 240
   Gln Phe Ser Lys Ser Leu Thr Asn Leu Lys Arg Ile Asp Leu Leu Pro
   245 250 255
   Tyr His Asn Tyr Gly Glu Asn Lys Tyr Gln Ala Ile Gly Arg Glu Tyr
   260 265 270
   Ser Leu Lys Glu Leu Lys Ser Pro Ser Lys Asp Lys Met Glu Arg Leu
   275 280 285
   Lys Ala Leu Val Glu Ile Met Gly Ile Pro Cys Thr Ile Gly Ala Glu
   290 295 300
<210> 8
   <211> 385
   <212> PRT
   <213> Clostridium butyricum
<400> 8
   Met Arg Met Tyr Asp Tyr Leu Val Pro Ser Val Asn Phe Met Gly Ala
   1 5 10 15
   Asn Ser Val Ser Val Val Gly Glu Arg Cys Lys Ile Leu Gly Gly Lys
   20 25 30
   Lys Ala Leu Ile Val Thr Asp Lys Phe Leu Lys Asp Met Glu Gly Gly
   35 40 45
   Ala Val Glu Leu Thr Val Lys Tyr Leu Lys Glu Ala Gly Leu Asp Val
   50 55 60
   Val Tyr Tyr Asp Gly Val Glu Pro Asn Pro Lys Asp Val Asn Val Ile
   65 70 75 80
   Glu Gly Leu Lys Ile Phe Lys Glu Glu Asn Cys Asp Met Ile Val Thr
   85 90 95
   Val Gly Gly Gly Ser Ser His Asp Cys Gly Lys Gly Ile Gly Ile Ala
   100 105 110
   Ala Thr His Glu Gly Asp Leu Tyr Asp Tyr Ala Gly Ile Glu Thr Leu
   115 120 125
   Val Asn Pro Leu Pro Pro Ile Val Ala Val Asn Thr Thr Ala Gly Thr
   130 135 140
   Ala Ser Glu Leu Thr Arg His Cys Val Leu Thr Asn Thr Lys Lys Lys
   145 150 155 160
   Ile Lys Phe Val Ile Val Ser Trp Arg Asn Leu Pro Leu Val Ser Ile
   165 170 175
   Asn Asp Pro Met Leu Met Val Lys Lys Pro Ala Gly Leu Thr Ala Ala
               180                 185                 190
   Thr Gly Met Asp Ala Leu Thr His Ala Ile Glu Ala Tyr Val Ser Lys
           195                 200                 205
   Asp Ala Asn Pro Val Thr Asp Ala Ser Ala Ile Gln Ala Ile Lys Leu
       210                 215                 220
   Ile Ser Gln Asn Leu Arg Gln Ala Val Ala Leu Gly Glu Asn Leu Glu
   225                 230                 235                 240
   Ala Arg Glu Asn Met Ala Tyr Ala Ser Leu Leu Ala Gly Met Ala Phe
                   245                 250                 255
   Asn Asn Ala Asn Leu Gly Tyr Val His Ala Met Ala His Gln Leu Gly
               260                 265                 270
   Gly Leu Tyr Asp Met Ala His Gly Val Ala Asn Ala Met Leu Leu Pro
           275                 280                 285
   His Val Glu Arg Tyr Asn Met Leu Ser Asn Pro Lys Lys Phe Ala Asp
       290                 295                 300
   Ile Ala Glu Phe Met Gly Glu Asn Ile Ser Gly Leu Ser Val Met Glu
   305                 310                 315                 320
   Ala Ala Glu Lys Ala Ile Asn Ala Met Phe Arg Leu Ser Glu Asp Val
                   325                 330                 335
   Gly Ile Pro Lys Ser Leu Lys Glu Met Gly Val Lys Gln Glu Asp Phe
               340                 345                 350
   Glu His Met Ala Glu Leu Ala Leu Leu Asp Gly Asn Ala Phe Ser Asn
   355 360 365
   Pro Arg Lys Gly Asn Ala Lys Asp Ile Ile Asn Ile Phe Lys Ala Ala
   370 375 380
   Tyr
   385
<210> 9
   <211> 35
   <212> ADN
   <213> Séquence artificielle
<220>
   <223> Description de la séquence artificielle:Amorce
<400> 9
   cgcggatccg tgattggagg agtaaaaatg ataag 35
<210> 10
   <211> 40
   <212> ADN
   <213> Séquence artificielle
<220>
   <223> Description de la séquence artificielle:Amorce
<400> 10
   tcccccgggg gaatccttta aatagtatta attaataagc 40

## Description

La présente invention concerne un procédé de préparation du 1,3-propanediol à partir d'une substance carbonée, ledit procédé comportant une étape de culture d'un micro-organisme recombinant non producteur de coenzyme B12 en l'absence d'ajout de coenzyme B12 ou de l'un de ses précurseurs.

L'invention concerne aussi un acide nucléique codant pour une glycérol deshydratase dont l'activité catalytique est indépendante de la présence de coenzyme B12 ou de l'un de ses précurseurs ainsi qu'un acide nucléique codant une 1,3-propanol déshydrogénase intervenant dans la synthèse du 1,3-propanediol.

L'invention est également relative à des vecteurs et cellules hôtes recombinants comprenant de tels acides nucléiques ainsi qu'aux polypeptides codés par ces derniers.

Le 1,3-propanediol est un composé d'un grand intérêt industriel, principalement mis en oeuvre dans l'industrie des détergents et dans celle des polymères.

Ainsi, le 1,3-propanediol est utilisé dans les lessives liquides en tant qu'agent stabilisant des lipases, amylases et protéases ainsi que comme « adoucissant protecteur » dans les détergents liquides pour lavage à la main de la vaisselle.

Par ailleurs, le 1,3-propanediol est utilisé de manière croissante dans l'industrie des polymères, plus particulièrement en tant que monomère de synthèse de polyesters, polyéthers, ou polyuréthanes.

Actuellement, la production de 1,3-propanediol est réalisée principalement par synthèse chimique par hydratation (en milieu acide) d'acroléine en 3-hydroxypiopionaldéhyde, lequel est ensuite réduit par hydrogénation catalytique en 1,3-propanediol.

Un tel procédé est coûteux et met en oeuvre un produit toxique, l'acroléine. De plus, cette synthèse est peu sélective et génère un grand nombre de composés secondaires non utilisables.

Une autre voie de synthèse consiste en l'hydrocarbonylation d'oxyde d'éthylène par le monoxyde de carbone et l'hydrogène sous haute pression en présence de catalyseurs et solvants.

Une telle réaction produit un dioxane qui est ensuite hydrogéné en 1,3-propanediol. Ce second procédé de synthèse chimique est également très coûteux.

Depuis quelques années, une alternative à la production de 1,3-propanediol par synthèse chimique a fait l'objet de différents travaux: il s'agit de la bioconversion du glycérol en 1,3-propanediol par certaines souches bactériennes telles que *Citrobacter, Clostridium, Enterobacter, Klebsiella, Lactobacillus* et *Pelobacter*.

En particulier, la conversion du glycérol en 1,3-propanediol chez les bactéries anaérobies facultatives telles que les bactéries du genre *Klebsiella,* du genre *Citrobacter* ou encore *Enterobacter agglomerans* a été étudiée.

Ainsi, des essais de clonage des gènes codant pour des enzymes responsables de la conversion du glycérol en 1,3-propanediol ont-ils été tentés à partir de la bactérie *Klebsiella pneumoniae*.

Plus particulièrement, le clonage des gènes codant pour deux enzymes a été recherché, respectivement une glycérol déshydratase qui catalyse la conversion du glycérol en 3-hydroxypropionaldéhyde, et une 1,3-propanediol déshydrogénase qui catalyse la conversion du glycérol en 3-hydroxypropionaldéhyde en 1,3-propanediol.

Les brevets US 5,633,362 et 5,821,092 décrivent le clonage d'un fragment génomique d'environ 35 kb de la bactérie *Klebsiella pneumoniae*, ledit fragment d'ADN contenant une séquence codant pour une diol déshydratase active. Un tel fragment génomique a été obtenu par criblage de plusieurs banques de cosmides des espèces bactériennes *Klebsiella pneumoniae* et *Klebsiella aerogenes*.

Il est décrit dans ces brevets que des souches de *E. Coli* DH5α ont été transformées avec ces cosmides et les bactéries transformantes criblées pour leur capacité à convertir le glycérol en 1,3-propanediol. Une faible production de 1,3-propanediol a été observée chez certains clones de bactéries transformantes. Il en a été déduit que la diol déshydratase codée par les cosmides sélectionnés pourrait être responsable de la conversion du glycérol en 1,3-propanediol observée.

Néanmoins, la production de 1,3-propanediol par les souches de *E. coli* DH5α transformées par les cosmides sélectionnés nécessitait obligatoirement la présence de vitamine B12 ou de l'un de ses précurseurs dans le milieu de culture bactérien.

De plus, la durée de fermentation nécessaire à la détection d'une production de 1,3-propanediol était très longue (de 78 à 132 heures) et le niveau de production du 1,3-propanediol très faible.

Le brevet US N°5,686,276 au nom de DU PONT DE NEMOURS & COMPANY décrit un procédé permettant la bioconversion du D-glucose en 1,3-propanediol par une souche de *E.coli* transformée par de l'ADN de cosmide originaire de *Klebsiella pneumoniae*. A nouveau, la production de 1,3-propanediol nécessite l'utilisation de milieux de culture adaptés aux cellules de *E. coli* transformée par un cosmide de *Klebsiella pneumoniae* contenant de la vitamine B12, par exemple à la concentration de 800µg/ml.

Les niveaux de production de 1,3-propanediol observés étaient très faibles, de l'ordre de 0,5 g/l à 10 g/l.

La présence de vitamine B12 dans le milieu de culture des cellules transformées avec l'ADN de *Klebsiella pneumoniae* des brevets US cités ci-dessus est obligatoire du fait que le coenzyme B12 est un cofacteur nécessaire à l'activité catalytique de la glycérol déshydratase de *Klebsiella pneumoniae*.

Le coenzyme B12, ou l'un quelconque de ces précurseurs, tel que la vitamine B12, est un composé excessivement coûteux et peu stable, ce qui rend difficile, voire impossible, la transposition à l'échelle industrielle des procédés de conversion du glucose ou du glycérol en 1,3-propanediol par fermentation bactérienne à l'aide de telles souches.

En outre, le coenzyme B12 et ses précurseurs traversent difficilement les parois membranaires de certains micro-organismes, telles que les levures, ce qui nécessite la présence de très hautes concentrations de ces composés dans le milieu de culture afin qu'ils soient accessibles aux enzymes intracellulaires dont le coenzyme B12 est le cofacteur.

Par ailleurs, une alternative qui consisterait à introduire l'ADN codant pour les glycérol déshydratases connues, intervenant dans la conversion du glycérol en 1,3-propanediol, dans des bactéries synthétisant la vitamine B12 se heurterait à des obstacles techniques considérables.

En effet, seules certaines espèces bactériennes synthétisent naturellement la vitamine B12, telles que les bactéries *Pseudomonas* ou encore les propionibactéries, dont la génétique est très peu connue et qui sont donc peu aptes à subir des modifications génétiques.

D'autres bactéries synthétisant naturellement la vitamine B12, dont la génétique est mieux connue, telle que *Klebsiella pneumoniae,* présentent d'importants problèmes de toxicité, ce qui les rend peu aptes à une utilisation dans l'industrie.

Outre ce premier désavantage, seule une faible production de 1,3-propanediol a pu être obtenue après transformation de *Klebsiella pneumoniae*. Ainsi, la demande PCT N°WO 98/21 339 décrit des *Klebsiella pneumoniae* recombinantes exprimant à la fois les gènes de métabolisation du glucose en glycérol et les gènes de métabolisation du glycérol en 1,3-propanediol. La production de 1,3-propanediol observée à partir de glucose était faible, de l'ordre de 10g par litre.

Les obstacles techniques à la mise au point d'un procédé de bioconversion d'une substance carbonée en 1,3-propanediol mentionnés ci-dessus, ont été surmontés par l'invention.

Le demandeur a en effet isolé et caractérisé un acide nucléique qui code pour une glycérol déshydratase dont l'activité catalytique est indépendante de la présence de coenzyme B12 ou de l'un quelconque de ces précurseurs.

Les gènes codant pour la glycérol déshydratase indépendante du coenzyme B12 ont été isolés par le demandeur à partir du génome de la bactérie *Clostridium butyricum* VPI 1718. Ils codent pour une protéine dimérique constituée de deux sous-unités protéiques, respectivement les polypeptides ORF11 et ORF12.

Il a été montré selon l'invention que les séquences codant pour les polypeptides ORF11 et ORF12 sont localisées sur un opéron unique dans le génome de *Clostridium butyricum*, ledit opéron comprenant également, en aval de la séquence nucléique codant pour la sous-unité ORF12, une région codant pour une 1,3-propanediol déshydrogénase.

L'opéron selon l'invention comprend, de l'extrémité 5' vers l'extrémité 3, un promoteur de la transcription , la séquence *orf11* codant pour ORF11, la première sous-unité de la glycérol déshydratase indépendante du coenzyme B12, la séquence *orf12*, codant pour ORF12, la seconde sous-unité de la glycérol déshydratase et une séquence *dhaT*, codant pour une nouvelle 1,3-propanediol déshydrogénase (DHAT).

Ainsi, les séquences nucléiques codant pour les deux sous-unités protéiques de la glycérol déshydratase et pour la 1,3-propanediol déshydrogénase font partie d'un opéron unique, les trois séquences codantes étant co-régulées par une séquence promotrice unique située du côté 5' de la séquence codant pour la sous-unité ORF11.

Il a en outre été montré selon l'invention que la transformation d'un hôte cellulaire bactérien avec une séquence comprenant les régions codant respectivement pour ORF11, ORF12 et DHAT était de nature à conférer à l'hôte cellulaire transformé la capacité de produire le 1,3-propanediol à partir du glucose ou du glycérol en l'absence du coenzyme B12 ou de l'un quelconque de ses précurseurs, lorsque ces séquences sont placées sous le contrôle d'un promoteur approprié, fonctionnel dans l'hôte cellulaire dans lequel l'expression de ces séquences est recherchée.

Le demandeur a également montré que la transformation d'un hôte cellulaire bactérien produisant naturellement le 1,3-propanediol par les séquences codant pour les deux sous-unités de la glycérol déshydratase selon l'invention était de nature à induire une augmentation significative de la production de propanediol chez les hôtes bactériens recombinants obtenus.

L'invention a donc pour objet un procédé de préparation du 1,3-propanediol à partir d'une substance carbonée, ledit procédé comportant au moins une étape de culture d'un micro-organisme recombinant dans lequel a été intégré au moins un acide nucléique codant pour les deux sous-unités d'une glycérol déshydratase dont l'activité catalytique est indépendante de la présence de coenzyme B12 ou l'un de ses précurseurs.

Avantageusement, la glycérol déshydratase dont l'activité catalytique est indépendante de la présence du coenzyme B12 provient de la bactérie *Clostridium butyricum*.

La glycérol déshydratase indépendante du coenzyme B12 est une protéine dimérique composée d'un premier polypeptide ayant au moins 50% d'identité en aminoacides avec le polypeptide de séquence SEQ ID N°6 et d'un second polypeptide ayant au moins 50% d'identité en aminoacides avec le polypeptide de séquence SEQ ID N°7. De manière tout à fait avantageuse, la glycérol déshydratase indépendante du coenzyme B12 est composée d'un premier et d'un second polypeptide ayant au moins 60%, 65%, 70%, 80%, 85%, 90%, 95% ou encore 99% d'identité en aminoacides avec respectivement le polypeptide de séquence SEQ ID N°6 et le polypeptide de séquence SEQ ID N°7.

Le « pourcentage d'identité » de nucléotides ou d'acides aminés entre deux séquences, au sens de la présente invention, peut être déterminé en comparant deux séquences alignées de manière optimale, à travers une fenêtre de comparaison.

La partie de la séquence nucléotidique ou peptidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal des deux séquences.

Le pourcentage d'identité est calculé en déterminant le nombre de positions auxquelles une base nucléique ou un acide aminé identique est observé pour les deux séquences comparées, puis en divisant le nombre de positions auxquelles il y a identité entre les deux bases ou les deux acides aminés, par le nombre total de positions dans la fenêtre de comparaison, puis en multipliant le résultat par cent afin d'obtenir le pourcentage d'identité de séquences.

L'alignement optimal des séquences pour la comparaison peut être réalisé de manière informatique à l'aide d'algorithmes connus (par exemple le logiciel FASTA de la Société WISCONSIN GENETICS SOFTWARE PACKAGE, GENETICS COMPUTER GROUP (GCG), 575 Science Doctor, Madison, WISCONSIN).

A titre d'illustration, le pourcentage d'identité de séquence pourra être calculé à l'aide du logiciel précité FASTA, en utilisant exclusivement les paramètres par défaut.

De manière tout à fait préférée, le pourcentage d'identité en acides nucléiques ou en acides aminés entre deux séquences nucléiques ou d'acides aminés est calculé à l'aide du logiciel BLAST (version 2.06 de Septembre 1998) en utilisant exclusivement les paramètres par défaut.

Les différences en acides aminés que peut comprendre un polypeptide selon l'invention par rapport à la séquence en acides aminés de référence, telle que définie dans le listage de séquences présenté à la fin de la présente description, peut résulter en des substitutions, délétions ou additions d'un ou plusieurs acides aminés consécutifs ou non.

Selon un autre aspect du procédé selon l'invention, le micro-organisme recombinant comprend en outre un acide nucléique codant une 1,3-propanediol déshydrogénase, de préférence une 1,3-propanediol déshydrogénase de *Clostridium butyricum*.

De manière préférée, la 1,3-propanediol déshydrogénase est un polypeptide ayant au moins 90% d'identité en aminoacides avec le polypeptide de séquence SEQ ID N°8.

De manière tout à fait avantageuse, l'invention concerne aussi un polypeptide ayant au moins 95%, 97%, 98% ou 99% d'identité en aminoacides avec le polypeptide de séquence SEQ ID N°8.

Une caractéristique essentielle du procédé selon l'invention est que l'étape de culture du micro-organisme recombinant est effectuée en l'absence de coenzyme B12 ou de l'un de ses précurseurs.

Selon encore un autre aspect, le procédé est en outre caractérisé en ce que la source carbonée est choisie parmi les osides et les polyols.

L'oside peut être par exemple le glucose.

Le polyol peut être par exemple le glycérol.

Préférentiellement, le procédé selon l'invention est mis en oeuvre avec un micro-organisme choisi parmi les micro-organismes ne produisant pas naturellement le coenzyme B12 ou l'un de ses précurseurs.

Un tel micro-organisme peut être une bactérie appartenant au genre *Clostridium* ou *Escherichia.*

Il peut s'agir également d'une levure de l'espèce *Saccharomyces cerevisiae*.

Selon un mode de réalisation particulier, le procédé est en outre caractérisé en ce que le micro-organisme recombinant comprend également des acides nucléiques codant respectivement une glycérol-3-phosphate déshydrogénase et une glycérol-3-phosphatase, auquel cas le micro-organisme recombinant est capable de convertir à haut rendement une source carbonée telle que le glucose en 1,3-propanediol.

L'invention a en outre pour objet un acide nucléique comprenant au moins 30 nucléotides consécutifs d'un polynucléotide codant pour au moine une sous-unité d'une glycérol déshydratase dont l'activité catalytique est indépendante de la présence de coenzyme B12 ou de l'un de ses précurseurs, ledit acide nucléique comprenant tout ou partie d'un polynucléotide de séquence SEQ ID N° 1 ou SEQ ID N° 2.

De préférence, un acide nucléique selon l'invention se présente sous une forme purifiée ou isolée.

Un polynucléotide de séquence complémentaire aux polynucléotides de séquences SEQ ID N°1 ou SEQ ID N°2 constitue également un objet de l'invention.

Font également partie de l'invention des acides nucléiques comprenant tout ou partie d'un polynucléotide possédant au moins 60%, 65%, 70%, 75%, 85%, 90%, 95%, 98%, 99%, 99,5% ou encore 99,8% d'identité en nucléotides avec la séquence nucléotidique de l'un quelconque des acides nucléiques dont les séquences sont définies dans la présente description, ou un acide nucléique de séquence complémentaire.

Le terme « isolé » au sens de la présente invention désigne un matériel biologique qui a été soustrait à son environnement originel (l'environnement dans lequel il est localisé naturellement).

Par exemple, un polynucléotide présent à l'état naturel dans une plante ou un animal n'est pas isolé.

Le même polynucléotide séparé des acides nucléiques adjacents au sein desquels il est naturellement inséré dans le génome de la plante ou de l'animal est isolé.

Un tel polynucléotide peut être inclus dans un vecteur et/ou un tel polynucléotide peut être inclus dans une composition et demeurer néanmoins à l'état isolé du fait que le vecteur ou la composition ne constitue pas son environnement naturel.

Le terme « purifié » ne nécessite pas que le matériel soit présent sous une forme de pureté absolue, exclusif de la présence d'autres composés. Il s'agit plutôt d'une définition relative.

Un polynucléotide est à l'état purifié après purification du matériel de départ ou du matériel naturel d'au moins un ordre de grandeur, de préférence deux ou trois et préférentiellement quatre ou cinq ordres de grandeur.

Aux fins de la présente description, l'expression « séquence nucléotique » peut être employée pour désigner indifféremment un polynucléotide ou un acide nucléique. L'expression « séquence nucléotidique » englobe le matériel génétique lui-même et n'est donc pas restreinte à l'information concernant sa séquence.

L'invention est également relative à un acide nucléique codant pour les deux sous-unités protéiques de la glycérol déshydratase indépendante du coenzyme B12 de *Clostridium butyricum*.

Préférentiellement, un tel acide nucléique est caractérisé en ce qu'il comprend un premier polynucléotide ayant au moins 50% d'identité en nucléotides avec le polynucléotide de séquence SEQ ID N°1 et un second polynucléotide ayant au moins 50% d'identité en nucléotides avec le polynucléotide de séquence SEQ ID N°2.

Selon un mode de réalisation particulier, un tel acide nucléique codant pour les deux sous-unités protéiques de la glycérol déshydratase indépendante du coenzyme B12 comprendra en outre une séquence à fonction de promoteur, fonctionnelle dans la cellule hôte dans laquelle l'expression des polynucléotides codant pour les deux sous-unités de cette enzyme est recherchée.

Une telle séquence nucléotidique promotrice de la transcription peut être la séquence promotrice SEQ ID N°3 ou une séquence ayant au moins 80% d'identité en nucléotides avec cette dernière.

L'invention a également pour objet un acide nucléique à fonction de promoteur bactérien, notamment chez *Clostridium butyricum*, comprenant un polynucléotide ayant au moins 80% d'identité en nucléotides avec la séquence SEQ ID N°3, ou un polynucléotide de séquence complémentaire.

Comme déjà mentionné plus haut, une organisation en opéron unique permet, chez la bactérie *Clostridium butyricum,* la synthèse d'un produit de transcription (ARN messager) comprenant à la fois les séquences codant pour les deux sous-unités protéiques de la glycérol déshydratase indépendante du coenzyme B12, qui catalyse la transformation du glycérol en 3-hydroxypropionaldéhyde (3-HPA) et la 1,3-propanediol déshydrogénase (DHAT) qui catalyse la transformation du 3-HPA en 1,3-propanediol.

L'invention concerne donc également un acide nucléique comprenant tout ou partie d'un polynucléotide codant une 1,3-propanediol déshydrogénase ayant au moins 90% d'identité en nucléotides avec le polynucléotide de séquence SEQ ID N°4, ou un polynucléotide séquence complémentaire.

Dans un mode de réalisation particulier d'un acide nucléique purifié ou isolé selon l'invention, il peut être avantageux de pouvoir inclure dans une même séquence les acides nucléiques codant respectivement pour les deux sous-unités protéiques de la glycérol déshydrogénase et pour la 1,3-propanediol déshydrogénase, un tel acide nucléique codant alors pour les deux enzymes susceptibles de catalyser la totalité des étapes de bioconversion du glycérol en 1,3-propanediol.

En conséquence, l'invention est également relative à un acide nucléique comprenant, de l'extrémité 5' vers l'extrémité 3':
a) un premier acide nucléique caractérisé en ce qu'il comprend un premier polynucléotide ayant au moins 50% d'identité en nucléotides avec le polynucléotide de séquence SEQ ID N°1 et un second polynucléotide ayant au moins 50% d'identité en nucléotides avec le polynucléotide de séquence SEQ ID N°2;
b) un second acide nucléique comprenant un polynucléotide codant une 1,3-propanediol déshydrogénase ayant au moins 90% d'identité en nucléotides avec le polynucléotide de séquence SEQ ID N°4.

Les séquences nucléotidiques codant respectivement pour la glycérol déshydratase et la 1,3-propanediol déshydrogénase de l'acide nucléique ci-dessus décrit, pourront en outre être avantageusement placées sous le contrôle d'une séquence promotrice appropriée, telle que la séquence SEQ ID N°3 ou de toute autre séquence promotrice fonctionnelle dans la cellule hôte dans laquelle leur expression est recherchée.

Un acide nucléique répondant à la définition ci-dessus est par exemple le polynucléotide de séquence SEQ ID N°5 ou encore un polynucléotide ayant au moins 50% d'identité en nucléotides avec le polynucléotide de séquence SEQ ID N°5.

L'acide nucléique de séquence SEQ ID N°5 comprend les éléments fonctionnels caractéristiques suivants:
a) un terminateur de transcription d'une région codante localisée en amont de l'opéron 1,3-propanediol dans le génome de *Clostridium butyricum*.
   Ce motif terminateur de transcription possède une structure en épingle à cheveux centré sur le nucléotide en position 27 de la séquence SEQ ID N°5 (ΔG = - 25,2 kcal/mol) qui comprend une tige 19 pb, les deux brins de la tige étant constitués respectivement des nucléotides en positions 5 à 23 et des nucléotides en positions 30 à 48 de la séquence SEQ ID N°5, la boucle de la structure en épingle à cheveux étant constituée par les nucléotides en positions 24 à 29 de la séquence SEQ ID N°5.
   Ce motif terminateur de transcription est suivi de la séquence ATTTT.
b) Promoteur de l'opéron 1,3-propanediol.
   Le promoteur de l'opéron 1,3-propanediol est localisé du nucléotide en position 100 au nucléotide en position 200 de la séquence SEQ ID N°5.
   Ce promoteur comprend une boîte (-35) de séquence TAGATA localisée du nucléotide en position 142 au nucléotide en position 147 de la séquence SEQ ID N°5. Ce promoteur comprend également une boîte (-10) de séquence TATTAT localisée du nucléotide en position 164 au nucléotide en position 169 de la séquence SEQ ID N°5, la distance entre les boîtes-35 et les boîtes -10 étant de 16 pb.
c) *orf11* (séquence codant pour la première sous-unité de la glycérol déshydrogénase.
   Il s'agit d'une phase de lecture ouverte unique de 2.361 pb codant pour le polypeptide ORF11 de 787 acides aminés de séquence SEQ ID N°6.
   Le codon d'initiation ATG est localisé du nucléotide en position 313 au nucléotide en position 315 de la séquence SEQ ID N°5. Le cadre ouvert de lecture se termine par un codon stop de séquence TAA localisée du nucléotide en position 2674 au nucléotide en position 2676 de la séquence SEQ ID N°5.
   En outre, un site de fixation au ribosome de séquence GAGGAG précède le codon d'initiation et est localisé du nucléotide en position 302 au nucléotide en position 307 de la séquence SEQ ID N°5.
d) *orf12* (séquence codant pour la seconde sous-unité de la glycérol déshydrogénase). Il s'agit d'un cadre de lecture ouvert unique de 912 pb codant pour un polypeptide de 304 acides aminés de séquence SEQ ID N°7. Le codon d'initiation de séquence ATG est localisé du nucléotide en position 2704 au nucléotide en position 2706 de la séquence SEQ ID N°5. Le cadre de lecture ouvert s'achève par un codon stop de séquence TAA localisée entre les nucléotides en position 3616 et le nucléotide en position 3618 de la séquence SEQ ID N°5.
   En outre, un site de fixation du ribosome de séquence AAGGGGA précède le codon d'initiation et est localisé du nucléotide en position 2689 au nucléotide en position 2695 de la séquence SEQ ID N°5.
e) *dhat* (séquence codant pour la 1,3-propanediol déshydrogénase).
   Il s'agit d'une phase de lecture ouverte unique de 1155 pb codant pour un polypeptide de 385 acides aminés de séquence SEQ ID N°8.
   Le codon d'initiation de séquence ATG est localisé du nucléotide en position 3678 au nucléotide en position 3680 de la séquence SEQ ID N°5
   La phase de lecture ouverte se termine par un codon stop de séquence TAA localisé du nucléotide en position 4833 au nucléotide en position 4835 de la séquence SEQ ID N°5.
   En outre, un site de fixation du ribosome de séquence AGGAGA précède le codon d'initiation et est localisé du nucléotide en position 3663 au nucléotide en position 3668 de la séquence SEQ ID N°5.
f) Terminateur de transcription de l'opéron 1,3-propanediol.
   Le terminateur de transcription de l'opéron 1,3-propanediol possède une structure en épingle à cheveux centrée sur le nucléotide en position 4933 de la séquence SEQ ID N°5 (ΔG = -27,4 kcal/mol) et comprend une tige de 22 pb constituée respectivement des nucléotides localisés en positions 4909 à 4930 de la séquence SEQ ID N°5 et des nucléotides localisés de la position 4936 à 4957 de la séquence SEQ ID N°5.

La boucle de l'épingle à cheveux est constituée de la séquence allant du nucléotide en position 4931 au nucléotide en position 4935 de la séquence SEQ ID N°5.

La structure en épingle à cheveux est suivie de la séquence TATTTTAATT.

Chacune des séquences fonctionnelles comprise dans l'opéron 1,3-propanediol de séquence SEQ ID N°5, tel que décrit ci-dessus, peut être mise en oeuvre individuellement, par exemple par insertion dans un vecteur de clonage et/ou d'expression; qu'il s'agisse de l'une des régions codant pour un polypeptide de l'invention ou encore d'une région régulatrice (promoteur ou terminateur de transcription).

De telles séquences nucléotidiques d'intérêt peuvent être obtenues selon des techniques bien connues de l'homme du métier telles que l'utilisation d'enzymes de restriction, dont l'utilisation est décrite en détail dans l'ouvrage de **SAMBROOK et al.** (1989) ou encore par amplification sélective de la séquence cible d'intérêt, par exemple par PCR.

Font également partie de l'invention les acides nucléiques hybridant, dans des conditions d'hybridation de forte stringence, avec un acide nucléique choisi parmi les séquences nucléotidiques SEQ ID N°1 à SEQ ID N°5.

Par « conditions d'hybridation de forte stringence » au sens de la présente invention, on entend les conditions d'hybridation suivantes:
- préhybridation des filtres pendant 8 heures à 65°C dans un tampon composé de 6 x SSC, 50 mM Tris-HCl (pH 7,5), 1 mM EDTA, 0,02% PVP, 0,02% FICOLL, 0,02% SAB et 500 µg/ml d'ADN de sperme de saumon dénaturé;
- hybridation des filtres pendant 48 heures à 65°C en présence de tampon 1 x SSC correspondant à 0,15 M de NaCl et 0,05 M de citrate de sodium;
- trois lavages des filtres dans une solution contenant un tampon 2 x SSC et 0,1% SDS à 68°C pendant 15'.

Les conditions de forte stringence définies ci-dessus sont adaptées à l'hybridation d'une molécule d'acide nucléique d'une longueur de 20 nucléotides.

Il va sans dire que ces conditions d'hybridation doivent être adaptées en fonction de la longueur de l'acide nucléique dont l'hybridation est recherchée, selon des techniques bien connues de l'homme du métier.

Les conditions convenables d'hybridation peuvent par exemple être adaptées selon l'enseignement contenu dans l'ouvrage de **HAMES & HIGGINS** (1985) ou encore dans l'ouvrage de **SAMBROOK et al**; (1989).

Font également partie de l'invention les acides nucléiques comprenant au moins 20 nucléotides consécutifs d'un polynucléotide choisi parmi les séquences nucléotidiques SEQ ID N°1 à SEQ ID N°5.

De tels acides nucléiques comprennent avantageusement 20, 25, 30, 35, 40, 50, 100, 150, 200 à 250, 300, 400, 500 nucléotides consécutifs d'un polynucléotide choisi parmi les séquences nucléotidiques SEQ ID N°1 à SEQ ID N°5.

De tels acides nucléiques peuvent comprendre par exemple 20, 25, 30, 35, 40, 50, 100, 150, 200, 250, 300, 400 ou 500 nucléotides consécutifs d'un polynucléotide choisi parmi les séquences nucléotidiques SEQ ID N°1 à SEQ ID N°5.

De tels acides nucléiques peuvent être notamment utiles en tant que sondes ou amorces nucléotidiques afin de détecter la présence de l'une quelconque des séquences nucléotidiques de SEQ ID N°1 à SEQ ID N°5 dans un échantillon.

De telles sondes ou amorces nucléotidiques peuvent être notamment utiles afin de mesurer l'expression de l'un quelconque des produits de transcription des régions codantes *orf11, orf12* ou *dhat* selon des techniques bien connues de l'homme du métier.

Afin d'améliorer encore la capacité des hôtes cellulaires transformés avec un acide nucléique selon l'invention à produire le 1,3 propanediol à partir de glucose, un tel hôte cellulaire recombinant pourra en outre être transformé avec un ou plusieurs gènes codant pour une ou plusieurs enzymes aptes à catalyser la transformation du glucose en glycérol.

Un couple d'enzymes capable d'effectuer la transformation du glucose en glycérol est par exemple une glycérol-3-phosphate déshydrogénase et une glycérol-3-phosphatase.

Ainsi, un acide nucléique selon l'invention pourra comprendre, outre les séquences codant respectivement pour la glycérol déshydratase indépendante du coenzyme B12 et la 1,3-propanediol déshydrogénase (dhaT), un troisième acide nucléique codant pour une glycérol-3-phosphate déshydrogénase et un quatrième acide nucléique codant pour une glycérol-3-phosphatase.

Pourront être employés en particulier un acide nucléique codant pour la gpd1 glycérol-3-phosphate déshydrogénase et un quatrième acide nucléique codant pour la gpp2 glycérol-3-phosphatase.

La gpd1 est par exemple décrite par **LARSSON et al.** (1993). Mol. Microbiol., 10, 1101-1111.

La gpd2 est par exemple décrite par **HIRAYAMA et al.** (1995). Mol. Gen. Genet., 249, 127-138.

Selon encore un autre aspect, l'invention concerne un vecteur recombinant de clonage et/ou d'expression comprenant un acide nucléique codant pour une glycérol déshydratase indépendante du coenzyme B12 ou une 1,3-propanediol déshydrogénase selon l'invention.

Un tel vecteur recombinant comprendra avantageusement une séquence promotrice , constitutive ou inductible, capable de diriger l'expression de la glycérol déshydratase indépendante du coenzyme B12 et/ou de la 1,3-propanediol déshydrogénase et un terminateur de transcription Rho-indépendant.

Il peut s'agir par exemple d'un vecteur navette capable de se répliquer dans différents hôtes cellulaires.

Un premier vecteur recombinant préféré selon l'invention est le plasmide pSPD5 contenu dans la souche de *Escherichia coli* déposée à la Collection National de Cultures de Microorganismes (CNCM) le 24 Juin 1999 sous le n° d'accès I-2243.

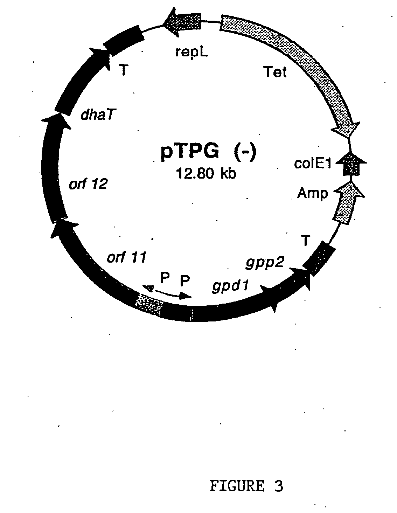

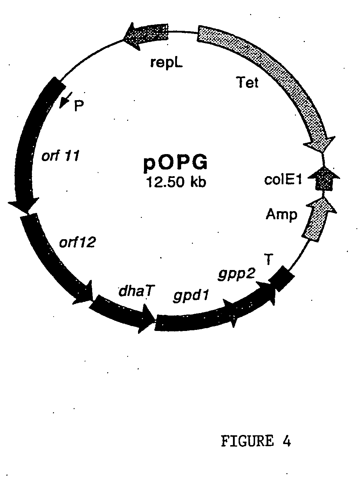

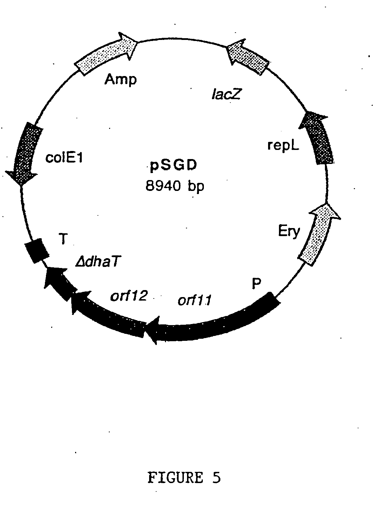

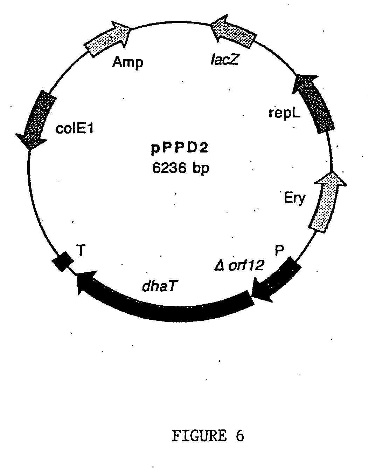

D'autres vecteurs préférés selon l'invention sont par exemple les suivants:
- les vecteurs pTPG(-) et pOPG représentés aux figures 3 et 4.
- Les vecteurs pSGD et pPPF2 représentés aux figures 5 et 6;
- des vecteurs possédant le réplicon du pCB101, tel que le vecteur pCTC511 (WILLIAMS et al., 1990) ou encore le vecteur pSYSL2 (**LEE et al**., 1992),
- un vecteur navette portant le réplicon pAMβ1 d'*Enterococus faecalis* DS-5, tel que le vecteur pCTC41 (**WILLIAMS et al**., 1990);
- les vecteurs navettes *E.coli B. Subtilis*/C. a*cetobutylicum* désignés pKNT11 et pKNT14 (**TRUFFAUT et al**., 1989);

L'invention concerne également une cellule hôte recombinante comprenant un acide nucléique ou un vecteur recombinant selon l'invention.

Une telle cellule hôte recombinante comprend avantageusement un acide nucléique codant pour la glycérol déshydratase indépendante du coenzyme B12 ou encore un vecteur contenant un tel acide nucléique.

De préférence, une telle cellule hôte recombinante comprendra un acide nucléique codant à la fois pour les deux sous-unités protéiques de la glycérol déshydratase indépendante du coenzyme B12 ainsi que pour la DHAT.

Il peut s'agir indifféremment d'une bactérie, d'un champignon ou d'une levure.

Une cellule hôte recombinante bactérienne sera choisie préférentiellement parmi *Escherichia coli, Clostridium* ou encore *Bacillus, lactobacillus et lactococcus*.

Une cellule de levure recombinante selon l'invention sera préférentiellement de la souche *Saccharomyces cerevisiae*.

Une cellule hôte recombinante préférée selon l'invention est la souche de *Escherichia coli* déposée à la Collection Nationale de Cultures de Microorganismes (CNCM) le 24 Juin 1999 sous le n° d'accès I-2243.

L'invention a également pour objet les polypeptides constituant respectivement l'une et l'autre des deux sous-unités protéiques constitutives de la glycérol déshydratase dimérique indépendante du coenzyme B12 selon l'invention.

De préférence, un polypeptide selon l'invention se présente sous une forme isolée ou purifiée.

L'invention se rapporte également à un polypeptide constituant l'enzyme 1,3-propanediol déshydrogénase de *Clostridium butyricum*.

Plus particulièrement, l'invention a trait à un polypeptide comprenant tout ou partie d'une séquence d'acides aminés ayant au moins 50% d'identité en acides aminés avec la séquence SEQ ID N°6 ou SEQ ID N°7.

L'invention concerne également une protéine dimérique composé d'un premier polypeptide ayant au moins 50% d'identité en aminoacides avec le polypeptide de séquence SEQ ID N°6 et d'un second polypeptide ayant au moins 50% d'identité en aminoacides avec le polypeptide de séquence SEQ ID N°7.

De manière tout à fait préférée, un tel polypeptide présente une activité catalytique glycérol déshydratase ne nécessitant pas la présence du coenzyme B12, une telle activité catalytique pouvant être mesurée conformément aux exemples.

Un autre objet de l'invention consiste en un polypeptide comprenant tout ou partie d'une séquence d'acides aminés ayant au moins 80% d'identité en acides aminés avec la séquence SEQ ID N°8.

De manière tout à fait préférée, un tel polypeptide présente une activité catalytique de 1,3-propanediol déshydrogénase.

L'invention est également relative à un procédé de production d'un polypeptide selon l'invention, caractérisé en ce qu'il comprend les étapes suivantes:
a) préparation d'un vecteur d'expression recombinant selon l'invention;
b) introduction du vecteur d'expression recombinant de l'étape a) dans une cellule hôte appropriée;
c) culture de la cellule hôte recombinante de l'étape b) dans un milieu de culture approprié;
d) récupération du polypeptide recombinant produit dans le surnageant de culture ou dans le lysat cellulaire;
e) le cas échéant, purification du polypeptide récupéré.

Les polypeptides selon l'invention peuvent être purifiés par exemple par passage sur une colonne de chromatographie d'affinité à ions nickel ou cuivre.

Ces polypeptides peuvent en outre être caractérisés par leur activité enzymatique glycérol déshydratase ou 1,3-propanediol déshydrogénase, comme indiqué dans les exemples.

Les polypeptides selon l'invention peuvent aussi être purifiés par exemple par chromatographique liquide à haute performance tels que des chromatographies HPLC en phase inverse et/ou d'échange cationique.

Font également partie de l'invention les polypeptides comprenant des modifications d'acides aminés allant de 1, 2, 3 , 4, 5, 10 à 20 substitutions, additions ou délétions d'un acide aminé par rapport à la séquence de l'une ou l'autre des deux sous-unités protéiques de la glycérol déshydratase indépendante du coenzyme B12 ou de la 1,3-propanediol déshydrogénase.

De manière tout à fait préférée, les modifications d'acides aminés dans un polypeptide de l'invention, par rapport aux polypeptides de référence, n'induiront pas de changement important de leur activité biologique. Ainsi, les modifications dans la séquence d'acides aminés des sous-unités protéiques de la glycérol déshydratase indépendante du coenzyme B12 selon l'invention seront tels que l'activité catalytique sera au moins égale à 50% de l'activité catalytique initiale et sera de préférence améliorée par rapport à l'activité catalytique initiale.

Il en est de même pour les modifications d'acides aminés dans la séquence protéique de la 1,3-propanediol déshydrogénase selon l'invention.

L'un quelconque des polypeptides selon l'invention ou encore un fragment peptidique de celui-ci peuvent être utilisés pour la préparation d'anticorps dirigés spécifiquement contre ce dernier.

Constitue également un objet de l'invention un polypeptide comprenant au moins 20 acides aminés consécutifs d'une séquence d'acides aminés choisie parmi les séquences SEQ ID N°6 à SEQ ID N°8.

Avantageusement, un tel polypeptide comprendra de 20, 25, 30, 35, 40, 45, 50, 75 à 100, 125, 150 ou 200 acides aminés consécutifs d'un polypeptide choisi parmi les séquences SEQ ID N°6 à SEQ ID N°8.

Préférentiellement, un tel polypeptide comprendra 20, 25, 30, 35, 40, 45, 50, 60, 70, 80, 90, 100, 125, 150, 175, 200, 250 ou 300 acides aminés consécutifs d'un polypeptide choisi parmi les séquences d'acides aminés SEQ ID N°6 à SEQ ID N°8.

De tels anticorps spécifiques peuvent être utilisés dans des tests d'immunodétection permettant de déterminer la présence d'une glycérol déshydratase indépendante du coenzyme B12 ou d'une 1,3-propanediol déshydrogénase dans un échantillon.

Un tel test d'immunodétection représente une alternative à la détermination de l'activité glycérol déshydratase ou 1,3-propanediol déshydrogénase dans un échantillon suspecté de contenir ces enzymes.

Par « anticorps » au sens de l'invention, on entendra les anticorps polyclonaux mais aussi des anticorps monoclonaux tels que préparés à partir d'hybridomes selon la technique décrite par **KOHLER & MILSTEIN** (1975).

Constituent également des anticorps au sens de la présente invention, des fragments d'anticorps (Fab', F(ab')₂ ainsi que des fragments d'anticorps simple chaîne Fv (brevet US N°4,946,778; **MARTINEAU et al**., (1998)) ou encore des anticorps humanisés (**REINMANN et al.,** 1997; **LEGER** et al. 1997).

L'invention sera en outre illustrée, sans pour autant être limitée, par les figures et les exemples suivants.

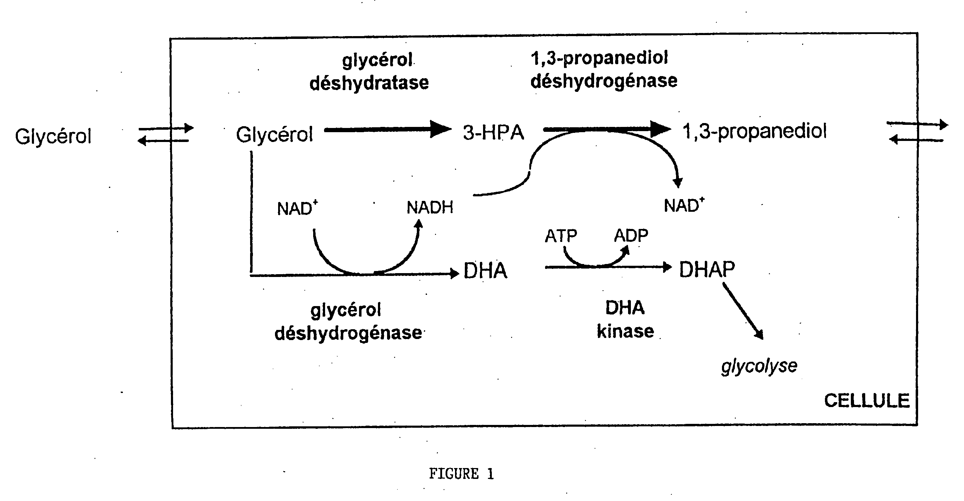

La figure **1** représente un schéma de la voie métabolique de bioconversion du glycérol en 1,3-propanediol chez la bactérie *Clostridium butyricum*.
La figure **2** illustre la construction du vecteur pSPD5 .
La figure **3** illustre la construction du vecteur pTPG (-).
La figure **4** illustre la construction du vecteur pOPG.
La figure **5** illustre la construction du vecteur pSGD.
La figure **6** illustre la construction du vecteur pPPD2.
La figure **7** illustre la production de 1,3-propanediol à partir du glycérol par la souche de *Clostridium acetobutylicum* DG1 (pSPD5).
La figure **8** illustre la production de 1,3-propanediol à partir du glycérol par la souche de *E. coli* DH5α transformée par le vecteur pSPD5 et cultivée en anaérobiose.
La figure **9** illustre la production de 1,3-propanediol à partir du glycérol par la souche non recombinée de *Clostridium butyricum* VPI 1718.
La figure **10** illustre la production de 1,3-propanediol à partir de glucose par la souche de *C-acetobutylicum* DG1 [pSPD5].

### MATERIELS ET METHODES.

### I. CULTURE DES SOUCHES

### 1. Souche utilisée

La souche de *Clostridium butyricum* à partir de laquelle ont été isolées et caractérisées les différentes séquences d'acides nucléiques selon l'invention est la souche VPI 1718.

La souche de *Clostridium acétobutylicum* utilisée pour la transformation avec les différents vecteurs décrits dans les exemples est la souche disponible à l'American Type Culture Collection sous le n°ATCC 824.

La souche de *Escherichia coli* DH5α utilisée pour la transfection avec les différents plasmides décrits dans les exemples est la souche disponible à LIFE TECHNOLOGIES Inc.

### 2. Les plasmides.

Le plasmide pUC18, d'une taille de 2,7kb est décrit par **YANNISCH-PERRON et al.** (1985).

Le plasmide pIMPI, d'une taille de 4,9 kb est décrit par **MERMELSTEIN** (1992).

Le plasmide pSOS95, d'une taille de 7 kb est décrit par **SOUCAILLE et PAPOUTSAKIS** (1996).

### 3. Milieux et conditions de culture

Pour la culture de la souche d'*Escherichia coli,* le milieu LB (décrit dans **SAMBROOK et al**., 1989) est supplémenté avec des antibiotiques (ampicilline à 100 µg/ml, érythromycine à 300 µg/ml, chloramphénicol à 35 µg/ml).

Les cultures des souches *Clostridium,* en particulier des souches de *Clostridium butyricum* ou des souches dé *Clostridium acetobutylicum* DGI transformées par le vecteur pSPD5 ont été réalisées dans le milieu suivant, dont la composition est donnée pour 1 litre de milieu de culture:

| | |
|---|---|
| extrait de levure | 4g |
| glycérol | 60g |
| KH₂PO₄ | 0,5g |
| K₂HPO₄ | 0,5g |
| MgSO₄ | 0,2g |
| NH₄Cl | 1,5g |
| FeSO₄ | 10 mg |
| biotine | 0,04 mg |
| acide paraaminobenzoïque | 8 mg |

Le pH est ajusté à 6 par addition d'ammonium et la température de croissance est de 37°C.

La clarithromycine est ajoutée à une concentration de 40 mg/l pour la culture de la souche *C. acetobutylicum* D61 (pSPP5).

### II. TECHNIQUES ANALYTIQUES.

### 1. Dosage des substrats et des produits.

L'ensemble des substrats (glucose et glycérol) et des produits (acétate, butyrate, lactate, 1,3-propanediol) sont dosés par chromatographie liquide à haute performance (CLHP).

L'appareil CLHP (pompe modèle 5810, Waters) est muni d'un passeur automatique (SP 8775, Spectra Physic.) possédant une boucle d'injection de 20 µl, d'un intégrateur (Intersmat ICR 1B, Shimadzu) et d'un réfractomètre (HP 1047A)

La séparation est obtenue par passage sur une colonne à exclusion d'ions (Aminex R HPX-87H, 300 mm x 7,8 mm, Biorad) équipée d'une précolonne (Micro-Guard, Biorad) garnie de la même résine ionique H⁺.

La phase mobile est constituée par de l'acide sulfurique à 0,031 mM, le flux est de 0,7 ml par minute et la séparation est réalisée à température ambiante.

### 2. Dosage des activités.

### a) Dosage de la 1,3-propanediol déshydrogénase.

Le dosage de la 1,3-propanediol déshydrogénase a été réalisé dans un volume de 1 ml du milieu réactionnel suivant:

| | |
|---|---|
| 1,3-propanediol | 100 mM |
| NAD⁺ | 2mM |
| DTT | 2 mM |
| (NH₄)₂SO₄ | 30 mM |
| K₂CO₃ | 100 mM |
| extrait cellulaire: essais sur 5 µl, 10 µl, 50 µl H₂O complété à | 1 ml |

La réduction du NAD⁺ est suivie par la mesure de la densité optique à la longueur d'onde de 340 nanomètres.

Le coefficient d'extinction ε(NADH) est de 6,22 mM⁻¹ x cm⁻¹.

### b) Dosage de la glycérol déshydratase

Le dosage de la glycérol déshydratase est réalisé sur un extrait cellulaire préalablement passé sur une colonne de désalage. Ce dosage est réalisé dans un volume 1 ml du milieu réactionnel suivant:

| | |
|---|---|
| KCl | 0,05 M |
| 1,2-propanediol | 0,06 M |
| tampon KPO₄ pH7 | 0,035 M |
| extrait cellulaire essai sur 5 µl, 10 µl, 20 µl, 30 µl. | |
| H₂O complété à | 1 ml |

La réaction est arrêtée après 10 minutes à 37 °C à l'aide de 1 ml de tampon citrate à 100 mM pH 3,6 et 500 µl de MBTH à 0,1%.

Après 15 minutes à 37°C, 1 ml d'eau est ajouté et le taux de propionaldéhyde formé est déterminé par mesure de la densité optique à la longueur d'onde de 305 nanomètres.

Le coefficient d'extinction molaire pour le produit formé est de 13,3 x 10³ M⁻¹ x cm⁻¹.

### c) Mesure de la biomasse

La croissance bactérienne est suivie par la mesure, dans des fractions aliquotes prélevées à des temps déterminés, de la densité optique à la longueur d'onde de 620 nanomètres pour un trajet optique de 1 cm. Il est considéré qu'une unité de densité optique correspond à environ 3x10⁸ bactéries/ml.

### III. Transformation des bactéries avec les vecteurs recombinants selon l'invention.

### III.1. Transformation d'E. coli DH5α

La souche d'*E. coli* DH5a est rendue compétente selon le protocole établi par **INOUE *et al*.** (1990). Ce protocole permet d'obtenir des efficacités de transformation de l'ordre de 10⁸ à 10⁹ transformants/mg de pUC18. Les cellules rendues compétentes sont ensuite conservées à -80°C. La transformation des cellules compétentes par un plasmide est réalisée par choc thermique (**SAMBROOK et al.,** 1989).

### III. 2. Transformation de C. acetobutylicum

Le plasmide à introduire dans *C. acetobutylicum* (ATCC 824 ou DG1) doit être préalablement méthylé au niveau des sites *Cac*824I (= *Bso*fI). La méthylation *in vivo* est réalisée en introduisant le plasmide à méthyler dans la souche *E. coli* ER 2275 portant le plasmide pAN1 (qui contient le gène codant pour la méthylase du phage f3TI de *Bacillus subtilis*). La préparation d'ADN plasmidique utilisée pour transformer *C*. *acetobutylicum* doit être très pure, purifiée par ultracentrifugation sur gradient de chlorure de césium (SAMBROOK et al., 1989) ou en utilisant la trousse Qiafilter Plasmid Midiprep (QIAGEN).

Les transformations sont réalisées en anaérobiose stricte selon le protocole suivant, adapté de celui de **MEMMERLSTEIN** (1992). Les efficacités sont encore très faibles, de l'ordre de 10² transformants par mg d'ADN.

A partir d'une préculture en milieu CGM (10ml) à 37°C sur une nuit,

Inoculer à 10% 50 ml de milieu 2YTG.

Arrêter la culture à DO600 de 1,0 à 1,2.

A partir de ce moment, l'ensemble des manipulations sont réalisées dans la hotte anaérobie et dans la glace.

Centrifuger les cellules pendant 10 min à 4000 g.

Laver le culot cellulaire dans 10 ml de tampon d'électroporation.

Centrifuger 10 min à 3000-4000 g.

Resuspendre les cellules dans 500 ml de tampon d'électroporation.

La suspension de cellules est mise en contact avec le plasmide (5 à 10 mg d'ADN plasmidique dissout dans 5 à 50 ml de tampon TE) préalablement introduit dans la cuvette d'électroporation (de 0,4cm d'épaisseur). Le tout est conservé dans la glace.

Le mélange est immédiatement soumis à une décharge électrique avec les paramètres suivants : V = 2500V, C = 25 mF, et R infinie (BioRad Gene pulser Il et Gene controller II) Dans ces conditions, le temps de la décharge délivrée varie de 7 à 12 ms.

Les cellules sont ensuite immédiatement transférées dans 10 ml de milieu 2 YTG et mises à incuber à 37°C jusqu'à reprise du métabolisme (formation de bulles de dioxyde de carbone et d'hydrogène).

La culture est alors centrifugée et le culot cellulaire repris dans un volume minimal du même milieu. La suspension est ensuite étalée sur milieu RCA avec antibiotique.

**Composition du tampon d'électroporation :**

| | |
|---|---|
| Saccharose | 270 mM |
| Tampon phosphate (Na2HPO4/NaH2PO4), pH 7.4 | 3 mM |

### EXEMPLE 1: Construction du vecteur d'expression pSPD5.

Les amorces nucléotidiques suivantes ont été synthétisées, afin d'amplifier une séquence nucléotidique contenant à la fois *orf11, orf12* et *dhaT,* c'est-à-dire l'ensemble des séquences codant à la fois pour les deux sous-unités protéiques de la glycérol déshydratase indépendante du coenzyme B12 et de la 1,3-propanediol déshydrogénase de *Clostridium butyricum*.

L'amorce PDH3 (SEQ ID N°9) inclut le site BamHI, le site de liaison aux ribosomes et le début de l'*orf11*.

L'amorce PDH4 (SEQ ID N°10), hybride avec le brin complémentaire et renferme le site Smal et la fin du gène dhaT.

Une réaction d'amplification avec les amorces pDH3 et pDH4 a été réalisée sur l'ADN génomique de *Clostridium butyricum* aux conditions suivantes:
- température d'appariement des amorces: 55°C;
- durée d'élongation: 4 minutes;
- nombre de cycles : 15 cycles.

La réaction d'amplification a été réalisée avec la trousse Expand Long Template PCR (commercialisée par Boehringer).

Cette réaction a permis d'amplifier un fragment de la taille attendue de 4,6 kb.

Ce fragment amplifié a été purifié sur gel d'agarose puis digéré par les enzymes BamHl et Smal.

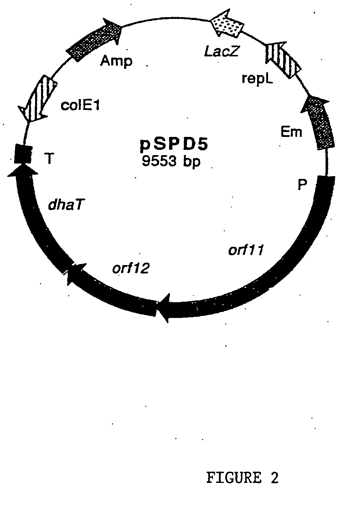

En parallèle, le fragment BamHI-Ehel de 4970 pb du vecteur navette *E.coli*/ *C*. *acetobutylicum* pSOS95 a été purifié sur gel d'agarose puis ligaturé avec le produit PCR de 4,6 kb. Le vecteur recombinant ainsi construit, appelé pSPD5 (figure 2), permet l'expression constitutive des gènes orf11, orf12 et dhaT sous lé contrôle du promoteur du gène de la thiolase de *C. acetobutylicum*.

### EXEMPLE 2: Construction des vecteurs d'expression pTPG(-) et pOPG

Les vecteurs pTPG(-) et pOPG sont des vecteurs dérivés du vecteur navette *E. coli-Clostridium* pTLH1 (***HARRIS et al***., 1999) qui permettent à une cellule hôte adéquate de réaliser la conversion directe du glucose en 1,3-propanediol. Essentiellement, ils permettent l'expression des gènes *orf*11, *orf*12 et *dha*T de *C. butyricum* (opéron 1,3-propanediol) ainsi que celle des gènes *GPD1* et *GPD2* de *S. cerevisae* codant respectivement pour la glycérol-3-phosphate déshydrogénase et la glycérol-3-phosphatase (opéron artificiel *gly*, porté par le plasmide pS2 (GELIS *et al*., 1999), dérivé du plasmide pSOS95, qui permet chez *E. coli* et *C*. *acetobutylicum* la conversion du glucose en glycérol). Ces deux plasmides se différencient par l'organisation de ces gènes, en deux opérons (1,3-propanediol et *gly*) dans pTPG(-), et sur un même opéron dans pOPG.

### 2.1. Construction de pTPG(-)

Dans un premier temps, ie fragment *Sal*l de 4,9 kpb du plasmide pSPD5 contenant la totalité de l'opéron 1,3-propanediol a été purifié sur gel d'agarose puis ligaturé au vecteur-navette pTLH1 digéré par *Sal*I, pour obtenir le plasmide pTLP de 10,6 kpb.

En parallèle, les amorces nucléotidiques OPGLY-D et OPGLY-R ont été synthétisées afin d'amplifier la totalité de l'opéron artificiel *gly*.

L'amorce OPGLY-D inclut le site *Sma*l ainsi que le début de l'opéron artificiel *gly*. Sa séquence est la suivante :
5'-GTTACCCGGGGCTCCTGCAGCTCGACTTTTTAAC-3'

L'amorce OPGLY-R hybride avec le brin complémentaire et inclut le site *Sma*l ainsi que la fin de l'opéron artificiel gly. Sa séquence est la suivante :
5'-TTTCACCCGGGAAACAGCTATGACCATGATTACG-3'

Une réaction d'amplification avec les amorces OPGLY-D et OPGLY-R a été réalisée sur le plasmide pS2, aux conditions suivantes :
- température d'appariement des amorces : 48°C
- durée d'élongation : 3, 5 min
- nombre de cycles : 10

La réaction a permis d'amplifier un fragment de la taille attendue de 2.2 kpb.

Ce fragment amplifié a été purifié sur gel d'agarose et digéré par l'enzyme *Sma*I, puis ligaturé au plasmide pTLP linéarisé par *Sma*l pour fournir le plasmide pTPG(-) de 12,8 kpb (Figure 3).

### 2.2. Construction de pOPG

Dans un premier temps, le fragment *Pvu*II*-Pst*I de 2,2 kpb du plasmide pS2 contenant la totalité de l'opéron glycérol a été purifié sur gel d'agarose puis ligaturé au fragment *Sma*I*-Pst*I de 5,7 kpb du vecteur-navette pTLH1, pour obtenir le plasmide pTLG1 de 7,9 kpb.

En parallèle, les amorces nucléotidiques dhaT-F et dhaT-R ont été synthétisées afin d'amplifier l'extrémité 5' du gène *dha*T.

L'amorce DHAT-F inclut le site *Bam*HI ainsi que la région centrale du gène *dha*T. Sa séquence est la suivante :
5'-TTGGATCCAGTATCTATAAATGATCCAATGC -3'

L'amorce DHAT-R hybride avec le brin complémentaire et inclut le site *Bgl*II ainsi que la fin du gène *dha*T. Sa séquence est la suivante :
5'-TTAGATCTTTTAAATAGTATTAATTAATAAGCAGCC -3'
Une réaction d'amplification avec les amorces dhaT-F et dhaT-R a été réalisée sur le plasmide pSPD5, aux conditions suivantes :
- température d'appariement des amorces : 48°C
- durée d'élongation : 1 min
- nombre de cycles : 10

La réaction a permis d'amplifier un fragment de la taille attendue de 0,65 kpb.

Ce fragment amplifié a été purifié sur gel d'agarose et digéré par les enzymes *Bam*HI et *Bgl*II, puis ligaturé au plasmide pTLG1 linéarisé par *Bam*HI pour fournir le plasmide pTPG de 8,55 kpb.

Dans un dernier temps, le fragment *Bam*HI-*Sbf*I de 4,0 kpb du plasmide pSPD5 a été purifié sur gel d'agarose puis ligaturé au fragment *Bam*HI-*Sbf*I de 8,55 kpb du plasmide pTPG pour obtenir le plasmide pOPG de 12,55 kpb (Figure 4).

### EXEMPLE 3: Construction du vecteur d'expression pSGD

Le vecteur pSGD est dérivé du plasmide pSPD5.

Essentiellement, le vecteur pSGD exprime fonctionnellement *orf11* et *orf12* (codant respectivement pour la première et la seconde sous-unités protéique de la glycérol déshydratase) et contient une délétion dans la région codant pour DHAT (1,3-propanediol déshydrogénase).

Le vecteur pSPD5 a été soumis à une digestion par l'enzyme de restriction Sbfl et le fragment de 4082 pb purifié sur gel d'agarose.

Il a ensuite été procédé à une digestion du vecteur pSOS95 par l'enzyme de restriction PstI et le fragment de 4858 pb purifié sur gel d'agarose.

Ces deux fragments ont ensuite été ligaturés afin d'obtenir le plasmide pSGD.

### EXEMPLE 4: Construction du vecteur d'expression pPPD2

Le vecteur pPPD2 est un vecteur dérivé du plasmide pSPD5 capable d'exprimer le gène *dhat* (codant pour la 1,3-propanediol déshydrogénase) et dans lequel *orf11* (codant pour la première sous-unité de la glycérol déshydratase) a été complètement délété ainsi que 100 pb à l'extrémité 5' de *orf12* (codant pour la seconde sous-unité de la glycérol déshydratase).

Le vecteur pSPD5 a tout d'abord été digéré simultanément par les enzymes de restriction BamHI et Mfel.

Le fragment BamHl-Mfel de 6326 pb obtenu par la double digestion à l'aide des endonucléases de restriction correspondantes a subi un traitement en présence de l'ADN polymérase T4 afin d'obtenir des extrémités franches.

Le fragment a alors subi une religation sur lui-même afin d'obtenir le plasmide pPPD2.

### EXEMPLE 5: Expression de l'opéron 1,3-propanediol dans Clostridium acetobutylicum DG1 cultivé sur glycérol.

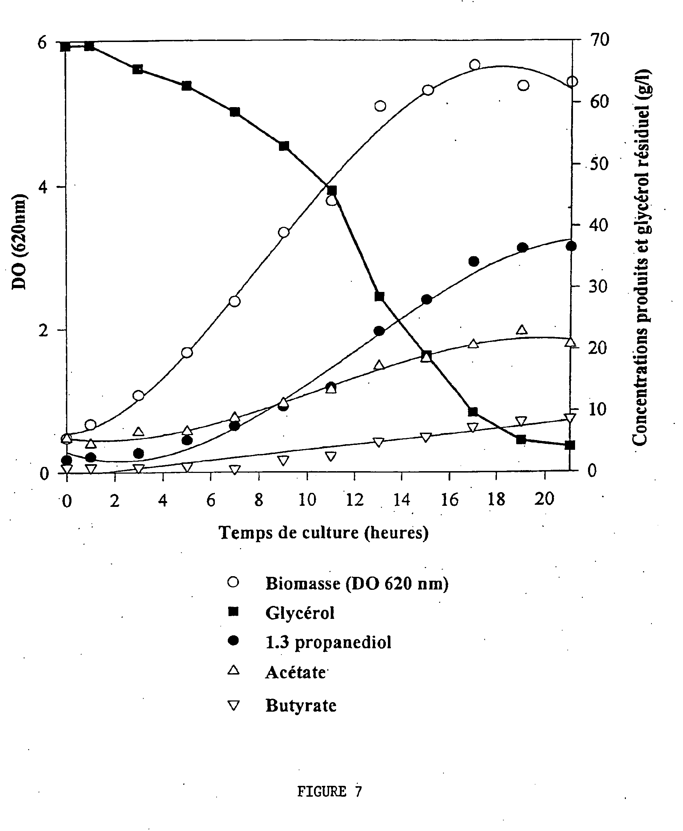

La souche de *Clostridium acétobutylicum* DGI transformée avec le plasmide pSPD5 comme décrit dans la partie Matériels et Méthodes a été mise en culture en présence de glycérol pendant des temps déterminés et différents paramètres ont été suivis au cours de la fermentation:
- la croissance bactérienne a été suivie par mesure de la densité optique à la longueur d'onde de 620 nanomètres et est représentée sur la figure 6 par les cercles ouverts;
- la concentration de glycérol a été suivie tout au long de la fermentation et est représentée sur la figure 7 par les carrés pleins;
- la synthèse de 1,3-propanediol a été également suivie tout au long de la fermentation et est représentée par les cercles pleins;
- de même la concentration d'acétate et de butyrate a été mesurée tout au long de la fermentation et est représentée sur la figure 7 respectivement par les triangles ouverts pointe en haut ou pointe en bas.

Les résultats présentés à la figure 7 montrent qu'une quantité significative de 1,3-propanediol est synthétisée par la souche de *Clostridium acetobutylicum* DGI transformée par le plasmide pSPD5 dès les quatre premières heures de culture. Au bout de 20 heures de fermentation, il peut être observée la production de 38 g/l de 1,3-propanediol. La production de 1,3-propanediol atteint un plateau aux environs de 18 heures après le début de la fermentation, ce plateau de production étant essentiellement dû au fait que la presque totalité du glycérol initial a été consommée par la bactérie transformée.

Il est important de noter que la souche *C. acetobutylicum* DG1 transformée par le plasmide de contrôle pIMP1 ne contenant pas les régions codant pour la glycérol déshydratase et la 1,3-propanediol déshydrogénase (**Memmerlstein**, 1992) ne se développe pas sur le milieu de culture avec du glycérol comme seule source carbonée et donc ne produit pas de 1,3-propanediol (Tableau 1).

### EXEMPLE 6: Expression de l'opéron 1,3-propanediol dans Escherichia coli DH5α cultivé sur glycérol.

Une souche d'*Escherichia coli* DH5α a été transformée avec le vecteur pSPD5, comme décrit dans la partie Matériels et Méthodes.

La souche d'*Escherichia coli* transformée avec le plasmide pSPD5 a été mise en culture en anaérobiose sur milieu LB supplémenté en glycérol (40 g/l) et en érythromycine (300µg/ml), la production de 1,3-propanediol a été mesurée à des temps déterminés.

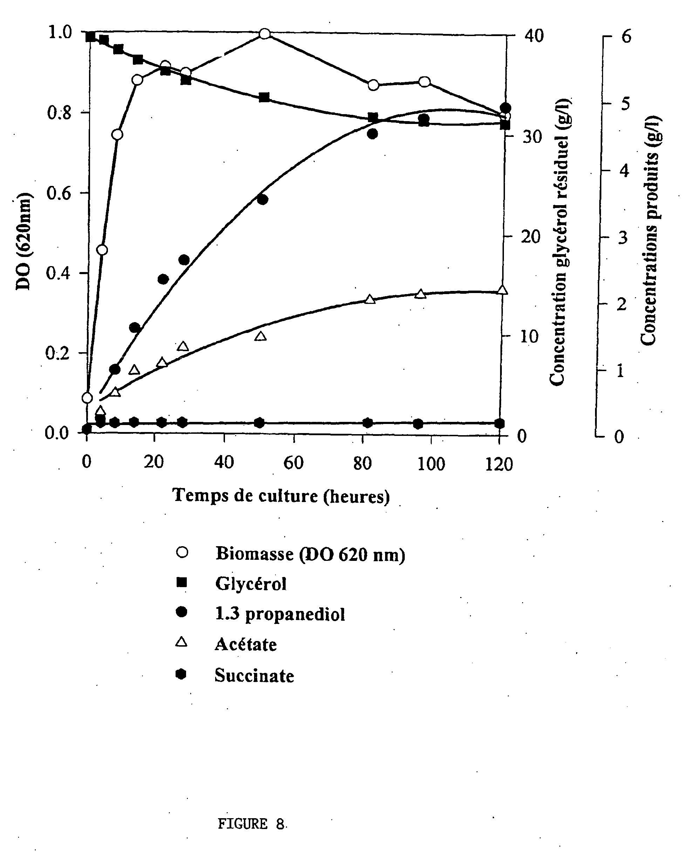

Les résultats sont rassemblés sur la figure 8.

Les résultats de la figure 8 montrent qu'une production significative de 1,3-propanediol est obtenue dès les premières heures de fermentation. Après 80 heures de fermentation, une production d'environ 4,5 g/l de 1,3-propanediol peut être observée.

La souche de contrôle *E. coli* DH5α[pIMP1] cultivée sur le même milieu ne conduit pas à la production de 1,3-propanediol (Tableau 1).

**TABLEAU 1 Production de 1,3-propanediol par des souches recombinantes cultivées à pH 6,5 en présence de glycérol dans des conditions anaérobies**

| | 1,3-propanediol (g/l) |
|---|---|
| *E. coli* DH5α (pIMP1) | 0 |
| *E. coli* DH5α (pSPDS) | 4,5 |
| *C. acetobutylicum* DG1 (pIMP1) | pas de croissance |
| *C. acetobutylicum* DG1 (pSPD5) | 38 |

Les résultats du tableau 1 montrent qu'une production significative de 1,3-propanediol (environ 5g/l) est obtenue avec la souche de *Escherichia coli* DH5α transfectée par le plasmide pSPD5, alors que la souche de *E.coli* transfectée avec le plasmide témoin pIMP1 ne produit pas de quantité détectable de 1,3-propanediol.

Avec la construction pSPD5, une quantité de 1,3-propanediol environ 7,6 fois supérieure est observée avec la souche *Clostridium acétobutylicum* par rapport à la souche de *Escherichia coli* DH5α. Ceci est due majoritairement au fait que les signaux de régulation du plasmide pSPD5 ont été optimisés pour l'expression de l'opéron 1,3-propanediol dans *Clostridium acetobutylicum*.

Des signaux de régulation, tels qu'un promoteur très actif chez *Escherichia coli* sont susceptibles de permettre l'obtention de quantité de 1,3-propanediol aussi importante chez *Escherichia coli* que celle observée chez *Clostridium acetobutylicum*.

### EXEMPLE 7: Expression de l'opéron 1,3-propanediol dans Clostridium butyricum sauvage cultivé sur glycérol.

La souche de *Clostridium butyricum* VPI 1718 a été mise en culture en présence de glycérol et la synthèse de 1,3-propanediol a été mesurée à des temps déterminés tout au long de la fermentation.

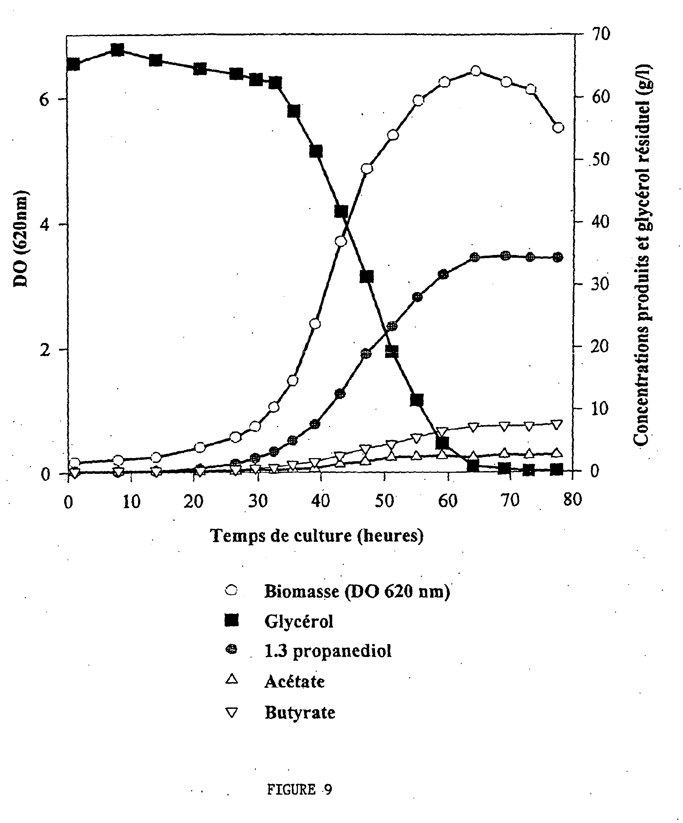

Les résultats sont représentés à la figure 9.

Les résultats de la figure 9 montrent qu'une production de 34 g/l de 1,3-propanediol est observée au bout de 60 heures de fermentation.

Si l'on se reporte aux résultats représentés sur la figure 7, on peut observer que la production de 1,3-propanediol par la souche de *Clostridium acetobutylicum* DG1 transformée par lé plasmide pSPD5 est beaucoup plus rapide (22 heures contre 60 heures) et supérieure à la production de 1,3-propanediol obtenue avec la souche de *Clostridium butyricum* exprimant naturellement la glycérol déshydratase indépendante du coenzyme B12 et la 1,3-propanediol déshydrogénase selon l'invention.

Par ailleurs, les niveaux d'activité catalytique, exprimés en unités par mg, de glycérol déshydratase et de 1,3-propanediol déhydrogénase ont été mesurés à la fois chez *Clostridium acetobutylicum* et chez *Clostridium butyricum*.

Les résultats sont représentés dans le tableau 2 DG1[pSPD5] ci-après.

**TABLEAU 2 Niveaux des enzymes impliquées dans la production de 1,3-propanediol dans les souches de C. acetobutylicum recombinantes et de C. butyricum**

| | **Glycérol déshydratase (U/mg)** | **1,3-propanediol déshydrogénase (U/mg)** |
|---|---|---|
| ***C.acetobutylicum* DG1 (PSPD5) croissance sur glycérol** | 3,5 | 2,4 |
| ***C.acetobutylicum*** **DG1 (pIMP1) croissance sur glucose** | 0,01< | 0,005< |
| ***C.butyricum* croissance sur glycérol** | 0,45 | 0,56 |

Les résultats présentés dans le tableau 2 indiquent que les niveaux d'activité de la glycérol déshydratase et de la 1,3-propanediol déshydrogénase exprimées dans la souche de *Clostridium acetobutylicum* transformée par le plasmide pSPD5 sont bien supérieurs (respectivement 7,8 et 4,3 fois supérieurs) aux niveaux d'activité glycérol déshydratase et 1,3-propanediol déshydrogénase exprimés par la souche de *Clostridium butyricum* produisant naturellement ces enzymes.

Comme contrôle, une souche de *Clostridium acétobutylicum* transformée avec le plasmide pIMPI ne contenant pas les régions codant pour la glycérol déshydratase et la 1,3-propanediol déshydrogénase ne produit pas de quantité détectable de ces deux enzymes, ce qui montre clairement que l'activité observée chez la souche transformée par le plasmide pSPD5 est uniquement due à l'expression de l'opéron 1,3-propanediol selon l'invention.

Les résultats présentés dans le tableau 2 démontrent clairement l'intérêt de transformer une souche de micro-organisme ne produisant pas naturellement la glycérol déshydratase et la 1,3-propanediol déshydrogénase selon l'invention avec un vecteur recombinant tel que le vecteur pSPD5 car il est possible dans ce cas, par exemple par l'emploi de promoteurs appropriés, d'obtenir des niveaux d'expression qui ne sont jamais atteints naturellement.

### EXEMPLE 8 ; expression des gènes de l'opéron 1,3-propanediol chez Saccharomyces cerevisia

### A. Matériels et Méthodes

### A.1 Matériels

**MILIEU MSL**

| SELS | g/l |
|---|---|
| -(NH₄)₂SO₄ | 5 |
| -MgSO₄, 7H₂O | 0,5 |
| -KH₂PO₄ | 1 |
| -NaCl | 0,1 |
| -CaCl₂, 2H₂O | 0,1 |

| **VITAMINES** | **mg/l** |
|---|---|
| -d-biotine | 0,02 |
| -Ca D(+)panthoténate | 0,4 |
| -myo-Inositol | 2,0 |
| -thiamine HCl | 0,4 |
| -pyridoxine HCl | 0,4 |
| -p-acide aminobenzoïque | 0,2 |
| -acide folique | 0,02 |
| -niacine | 0,4 |
| -riboflavine | 0,2 |

| **OLIGO-ELEMENTS** | **mg/l** |
|---|---|
| -ZnSO₄, 7H₂O | 0,4 |
| -MnSO₄ | 0,4 |
| -CuSO_{4,} 5H₂O | 0,04 |
| -Na₂MoO₄,2H₂O | 0,2 |
| -FeCl₃, 6H₂O | 0,2 |
| -H₃BO₃ | 0,5 |
| KI | 0,1 |

| **SOURCE DE CARBONE** | **g/l** |
|---|---|
| - glucose | 10 |
| - glycérol | 10 |

| **AA (drop-out)** | **g/l** |
|---|---|
| -L Arg | 0,02 |
| -Thre | 0,05 |
| -L Tryp | 0,04 |
| -L Isoleu | 0,06 |
| -Lys | 0,04. |
| -Met | 0,01 |
| -Phe | 0,06 |
| -Tyr | 0,05 |
| -Adénine | 0,01 |
| -Ergostérol | 0,01 |
| -Tween 80 | 0,42 |

### A.2 Méthodes

Les gènes *orf11, orf12* et *dhat* ont été individuellement amplifiés par PCR en introduisant grâce aux amorces un site *SmaI* à l'extrémité 5' et un site *ApaI* à l'extrémité 3'. Chaque gène a ensuite été introduit, grâce à ces deux sites de restriction, dans le vecteur pYGK (CROUX et SOUCAILLE, 1999) permettant de construire une cassette d'expression sous le contrôle du promoteur du gène *PGK* et du terminateur du gène *CYC1*. Les trois gènes dans leur cassette d'expression ont ensuite été sortis de chacun des vecteurs obtenus précédemment (pYGK11, pYGK12 et pYGKT) par digestion *NotI-SacII* introduits dans trois vecteurs multimériques de la famille pRS42x qui diffèrent par la nature de leur marqueur de sélection (*HIS3* pour pRS423, *LEU2* pour pRS425 et *URA3* pour pRS426) préalablement digérés par les mêmes enzymes de restriction. Chacun des trois plasmides obtenus (pRSGK11, pRSGK12 et pRSGKT) a ensuite été introduit dans la souche *S*. *cerevisae* JF624 (*leu2 ura3 lys2 trp1 his3)* et sélectionné par complémentation des 3 auxotrophies de cette souche. La souche *S. cerevisae* JF624 (pRSGK11, pRSGK12, pRSGKT) et la souche de contrôle *S. cerevisae* JF624 (PRS423, PRS425, pRS426) ont ensuite été cultivées en anaérobiose pendant 36 heures dans le milieu MSL.

### B. RESULTATS

Les résultats de l'expression des gènes de l'opéron 1,3 propanediol dont les souches de *S*. *cerevisiae* JF624 décrits au paragraphe A.2 ci-dessus sont présentés dans le tableau 3 ci-dessous.

Les résultats du tableau 3 montrent que la souche *S. cerevisiae* JF624 qui contient les inserts des gènes *orf11, orf12* et *dhat* insérés respectivement dans les plasmides pRSGK11, pRSGK12 et pRSGKT est capable de produire le 1,3 propanediol à partir d'une source de glycérol sans addition de vitamine B12.

**TABLEAU 3**

| | Ethanol (g/l) | 1,3-propanediol (g/l) |
|---|---|---|
| S. cerevisae JF624 (pRS423, pRS425,pRS426 | 4,5 | 0 |
| S. cerevisae JF624 (pRSGK11, pRSGK12, pRSGKT) | 4,8 | 0,1 |

### EXEMPLE 9: Expression de l'opéron 1,3-propanediol dans Clostridium acetobutylicum DG1 [pSPD5] cultivé sur glucose

La souche *Clostridium acetobutylicum* DG1 transformée par le plasmide pSPD5 comme décrit dans la partie Matériels et Méthodes a été cultivée en présence de glucose comme seule source carbonée et différents paramètres ont été suivis au cours de la fermentation.

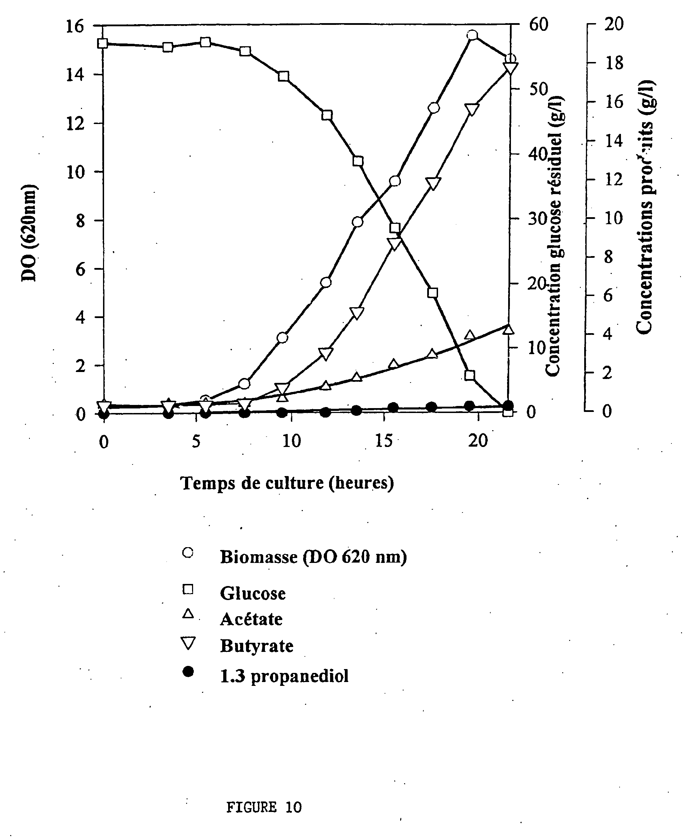

Les résultats sont représentés sur la figure 10.

Les résultats de la figure 10 montrent une synthèse, quoique faible (0,3 g/l) de 1,3-propanediol, alors que la totalité du glucose a été consommée. Cette production, due à la présence d'une faible concentration en glycérol intracellulaire chez *Clostridium acetobutylicum* DG1 [pSPD5] démontre toutefois la faisabilité d'un procédé de conversion directe du glucose en 1,3-propanediol dans une souche portant les gènes revendiqués par la présente invention.

### EXEMPLE 10

### Expression de l'opéron 1,3-propanediol dans Bacillus subtilis 168 [pSPD5] cultivée sur glycérol.

La souche *Bacillus subtilis* 168 transformée par ie plasmide pSPD5 comme décrit dans la partie Matériels et Méthodes, a été cultivée sur milieu LB + 10 g/l glycérol + 20 mM nitrate + 2µg/ml érythromycine en anaérobiose et comparée à la même souche transformée par leplasmide de contrôle pIMP1.

Les résultats sont représentés dans le tableau 4 ci-après.

**TABLEAU 4**

| | Acétate (g/l) | 2,3-butanediol (g/l) | 1,3-propanediol (g/l) |
|---|---|---|---|
| B.subtilis 168 (pSPD5) | 0,84 | 1,2 | 0 |
| B. subtilis 168 (pSPD5) | 0,9 | 1,0 | 0,2 |

Les résultats du tableau 3 montrent que l'expression de l'opéron 1,3 propanediol dans *B. subtilis* permet la production de 1,3-propanediol à partir de glycérol sans ajout de vitamine B12.

### EXEMPLE 11: Conversion du glucose en 1,3-propanediol par E.coli NZN 111 [pTPG].

Le plasmide pTPG(-) et le plasmide de contrôle pTLH1 ont été introduits dans la souche *E. coli* NZN 111 (*pfl:cat, ldh:kan*), une souche incapable de produire du formate et du lactate en anaérobiose. Ces deux souches recombinantes ont ensuite été cultivées en anaérobiose pendant 48 heures dans le milieu LB + glucose + 10 µg/ml de tétracycline sans régulation de pH. Les résultats présentés dans le tableau 5 montrent que *E. coli* peut produire des quantités significatives de 1,3-propanediol à partir de glucose après transformation par le plasmide PTPG.

**TABLEAU 5**

| | Acétate g/l | Succinate (g/l) | Glycérol (g/l) | 1,3 propanediol (g/l |
|---|---|---|---|---|
| *E.coli* NZN111 (pTLH1) | 0,15 | 0,4 | 0 | 0 |
| *E.coli* NZN111(pTPG) | 1,25 | 0,2 | 2,1 | 0,7 |

### REFERENCES BIBLIOGRAPHIQUES:

- **Gelis, C. et al.,** 1999 - Non publié.
- **Hames BD and Higgins SJ,** 1985, "Nucleic acid hybridization : a practical approach", Hames and Higgins Ed., IRL Press, Oxford.
- **Harris, L.M. et al.,** 1999 - Non publié.
- **Houbenweyl,** 1974, in Methode der Organischen Chemie, E. Wunsch Ed., Volume 15-I et 15-II,
- **Inoue et al.,** 1990, Gene, 96, 23-28.
- **Kohler G. and Milstein C.,** 1975, Nature, **256 :** 495.
- **Lee S.Y. et al.,** 1992, Biotech Lett., **14 :** 427-432.
- **Leger OJ, et al.**, 1997, Hum Antibodies, **8**(1): 3-16
- **Maguin E. et al.**, 1992, J. Bacteriol., **174 :** 5633-5638.
- **Martineau P, Jones P, Winter G,** 1998, J Mol Biol, **280**(1):117-127
- **Memmerlstein D.L.**, 1992, PhD Thesis, Northwestern University, Evanston, Illinois, USA.
- **Merrifield RB,** 1965a, Nature, **207**(996): 522-523.
- **Merrifield RB.,** 1965b, Science, **150**(693): 178-185.
- **Reimann KA, et al**., 1997, AIDS Res Hum Retroviruses. **13**(11): 933-943
- **Sambrook, J. Fritsch, E. F., and T. Maniatis**. 1989. Molecular cloning: a laboratory manual. 2ed.
- **Soucaille P. et Papoutsakis,** 1996, **Non Publié.**
- **Truffaut N. et al.,** 1989, FEMS Microbiol. Lett., **58 :** 15-20.
- **Williams D.R. et al.,** 1990, J. Gen. Microbiol., **136 :** 819-826
- **Yannish-Perron C. et al.,** 1985, Gene, **33 :** 103-119.

## Revendications

1. Procédé de préparation du 1,3-propanediol à partir d'une substance carbonée, ledit procédé comportant au moins une étape de culture d'un micro-organisme recombinant dans lequel a été introduit au moins un acide nucléique codant pour les deux sous-unités d'une glycérol déshydratase dont l'activité catalytique est indépendante de la présence de co-enzyme B12 ou de l'un de ses précurseurs, cette protéine dimérique étant composée d'un premier polypeptide ayant au moins 50% d'identité en amino acides avec le polypeptide de séquence SEQ ID N°6 et d'un second polypeptide ayant au moins 50% d'identité en amino acides avec le polypeptide de séquence SEQ ID NO 7.

2. Procédé selon la revendication 1, **caractérisé en ce que** la glycérol déshydratase dont l'activité catalytique est indépendante de la présence de co-enzyme B12 ou de l'un de ses précurseurs est originaire de *Clostridium butyricum*.

3. Procédé selon l'une des revendications 1 et 2, **caractérisé en ce que** le micro-organisme recombinant comprend en outre un acide nucléique codant une 1,3-propanediol déshydrogénase, et de manière préférée une 1,3-propanediol déhydrogénase de *Clostridium butyricum* VPI 1718.

4. Procédé selon la revendication 3, **caractérisé en ce que** la 1,3-propanediol déhydrogénase est un polypeptide ayant au moins 90% d'identité en amino acides avec le polypeptide de séquence SEQ ID NO 8.

5. Procédé salon l'une des revendications 1 à 4, **caractérisé en ce que** l'étape de culture du micro-organisme recombinant est effectuée en l'absence de co-enzyme B12 ou de l'un de ses précurseurs.

6. Procédé selon l'une des revendications 1 à 5, **caractérisé en ce que** la substance carbonée est choisie parmi les osides et les polyols.

7. Procédé selon la revendication 6, **caractérisé en ce que** l'oside est le glucose et le polyol est le glycérol.

8. Procédé selon l'une des revendications 1 à 7, **caractérisé en ce que** le micro-organisme est choisi parmi les micro-organismes ne produisant pas naturellement le co-enzyme B12 ou l'un de ses précurseurs.

9. Procédé selon la revendication 8, **caractérisé en ce qu'**il s'agit d'une bactérie, d'une levure ou d'un champignon.

10. Procédé selon la revendication 9, **caractérisée en ce qu'**il s'agit d'une bactérie appartenant au genre *Clostridium, Escherichia, Bacillus, Lactobacillus ou Lactococcus*.

11. Procédé selon la revendication 9, **caractérisé en ce qu**'il s'agit de la levure *Saccharomyces cerevisiae*.

12. Procédé selon l'une des revendications 1 à 11, **caractérisé en ce que** le micro-organisme recombinant comprend également des acides nucléiques codant respectivement pour une glycérol-3-phosphate déhydrogénase et une glycérol-3-phosphatase.

13. Acide nucléique comprenant au moins 30 nucléotides consécutifs d'un polynucléotide codant pour au moins une sous-unité d'une glycérol déshydratase dont l'activité catalytique est indépendante de la présence de co-enzyme B12 ou de l'un de ses précurseurs, ledit acide nucléique comprenant tout ou partie d'un polynucléotide ayant au moins 50% d'identité en nucléotides avec le polynucléotide de séquence SEQ ID NO 1, ou SEQ ID N°2 ou un polynucléotide de séquence complémentaire.

14. Acide nucléique selon la revendication 13, **caractérisé en ce qu'**il comprend un premier polynucléotide ayant au moins 50% d'identité en nucléotides avec le polynucléotide de séquence SEQ ID N°1 et un second polynucléotide ayant au moins 50% d'identité en nucléotides avec le polynucléotide de séquence SEQ ID N°2.

15. Acide nucléique selon la revendication 14, **caractérisé en ce qu'**il comprend en outre une séquence à fonction de promoteur de la transcription, fonctionnelle dans la cellule hôte dans laquelle l'expression du polynucléotide est recherchée.

16. Acide nucléique selon la revendication 15, **caractérisé en ce que** la séquence promotrice est la séquence SEQ ID N0 3 ou une séquence ayant au moins 80 % d'identité en nucléotides avec cette dernière.

17. Acide nucléique à fonction de promoteur bactérien comprenant un polynucléotide ayant au moins 80% d'identité en nucléotides avec la séquence SEQ ID NO 3, ou un polynucléotide de séquence complémentaire.

18. Acide nucléique comprenant, de l'extrémité 5' vers l'extrémité 3', un premier acide nucléique selon la revendication 14 et un second acide nucléique comprenant tout ou partie d'un polynucléotide codant pour une 1,3-propanediol déshydrogénase ayant au moins 90% d'identité en nucléotides avec le polynucléotide de séquence SEQ ID N°4.

19. Acide nucléique selon la revendication 18, **caractérisé en ce qu'**il s'agit d'un polynucléotide ayant au moins 50% d'identité en nucléotides avec la séquence nucléotidique SEQ ID N° 5.

20. Acide nucléique selon la revendication 18, **caractérisé en ce qu'**il comprend en outre un troisième acide nucléique codant une glycérol-3-phosphate déshydrogénase et un quatrième acide nucléique codant une glycérol-3-phosphatase.

21. Vecteur de clonage et/ou d'expression recombinant comprenant un acide nucléique selon l'une des revendications 14 à 20.

22. Vecteur recombinant selon la revendication 21, **caractérisé en ce qu'**il s'agit du plasmide pSPD5 contenu dans la souche de *Escherichia coli* déposée à la Collection Nationale de Cultures de Micro-organismes (CNCM) le 24 Juin1999 sous le N° d'accès I-2243.

23. Cellule hôte recombinante comprenant un acide nucléique selon l'une des revendications 14 à 20 ou un vecteur recombinant selon l'une des revendications 21 et 22.

24. Cellule hôte recombinante selon la revendication 23, **caractérisée en ce qu'**il s'agit de la souche de *Escherichla coli* déposée à la Collection Nationale de Cultures de Micro-organismes (CNCM) le 24 Juin 1999 sous le N° d'accès I-2243.

25. Polypeptide codé par un acide nucléique selon l'une des revendications 13 et 14, comprenant au moins 20 acides aminés consécutifs d'une séquence d'acides aminés ayant au moins 50% d'identité en acides aminés avec la séquence SEQ ID NO 6 ou SEQ ID N°7.

26. Protéine dimérique composée d'un premier polypeptide ayant au moins 50% d'identité en amino-acides avec le polypeptide de séquence SEQ ID N°6 et d'un second polypeptide ayant au moins 50% d'identité en amino-acides avec le polypeptide de séquence SEQ ID N°7.

27. Procédé de production d'un polypeptide selon l'une des revendications 25 et 26, **caractérisé en ce qu'**il comprend les étapes suivantes:
a) préparation d'un vecteur d'expression recombinant selon l'une des revendications 21 et 22;
b) introduction du vecteur d'expression recombinant de l'étape a) dans une cellule hôte appropriée;
c) culture de la cellule hôte recombinante de l'étape b) dans un milieu de culture approprié;
d) récupération du polypeptide recombinant produit dans le surnageant de culture ou dans le lysat cellulaire;
e) le cas échéant, purification du polypeptide.

28. Anticorps dirigé spécifiquement contre un polypeptide choisi parmi les polypeptides de séquence en acides aminés SEQ ID n°6 ou SEQ ID n°7.

## Claims

1. Process for the preparation of 1,3-propanediol from a carbon-containing substance, said process comprising at least one step which consists of growing a recombinant micro-organism in which has been introduced at least one nucleic acid coding for the two subunits of a glycerol dehydratase the catalytic activity of which is independent of the presence of coenzyme B12 or one of its precursors, said dimeric protein being composed of a first polypeptide having at least 50 % amino acid identity with the polypeptide of sequence SEQ ID No.6 and a second polypeptide having at least 50 % amino acid identity with the polypeptide of sequence SEQ ID No.7.

2. Process according to Claim 1, **characterized in that** the glycerol dehydratase the catalytic activity of which is independent of the presence of coenzyme B12 or one of its precursors is derived from *Clostridium butyricum*.

3. Process according to one of Claims 1 and 2, **characterized in that** the recombinant micro-organisin comprises in addition a nucleic acid coding for a 1,3-propanediol dehydrogenase and preferably a 1,3-propanediol dehydrogenase of *Clostridium butyricum* VPI 1718.

4. Process according to Claim 3, **characterized in that** the 1,3-propanediol dehydrogenase is a polypeptide having at least 90 % amino acid identity with the polypeptide of sequence SEQ ID No. 8.

5. Process according to one of Claims 1 to 4, **characterized in that** the culture step of the recombinant micro-organism is carried out in the absence of coenzyme B12 or one of its precursors.

6. Process according to one of Claims 1 to 5, **characterized in that** the carbon-containing substance is selected from the carbohydrates and the polyols.

7. Process according to Claim 6, **characterized in that** the carbohydrate is glucose and the polyol is glycerol.

8. Process according to one of Claims 1 to 7, **characterized in that** the micro-organism is selected from the micro-organisms not producing coenzyme B12 or one of its precursors naturally.

9. Process according to Claim 8, **characterized in that** it is a bacterium, a yeast or a fungus.

10. Process according to Claim 9, **characterized in that** it is a bacterium belonging to the *Clostridium, Escherichia, Bacillus, Lactobacillus* or *Lactococcus* genus.

11. Process according to Claim 9, **characterized in that** it is the yeast *Saccharomyces cerevisiae*.

12. Process according to one of Claims 1 to 11, **characterized in that** the recombinant micro-organism also comprises nucleic acids coding for a glycerol-3-phosphate dehydrogenase and a glycerol-3-phosphatase.

13. Nucleic acid comprising at least 30 consecutive nucleotides all or part of a polynucleotide coding for at least one subunit of a glycerol dehydratase, the catalytic activity of which is independent of the presence of coenzyme B12 or one of its precursors, said nucleic acids comprising all or part of a polynucleotide having at least 50 % nucleotide identity with the polynucleotide of sequence SEQ ID No. 1 or SEQ ID No.2 or a polynucleotide with a complementary sequence.

14. Nucleic acid according to Claim 13, **characterized in that** it comprises a first polynucleotide having at least 50 % nucleotide identity with the polynucleotide of sequence SEQ ID No.1 and a second polynucleotide having at least 50 % nucleotide identity with the polynucleotide of sequence SEQ ID No.2.

15. Nucleic acid according to Claim 14, **characterized in that** it comprises in addition a sequence with a transcription promoter function, functional in the host cell in which the expression of the polynucleotide is desired.

16. Nucleic acid according to Claim 15, **characterized in that** the promoter sequence is the sequence SEQ ID No.3 or a sequence having at least 80 % nucleotide identity with this latter.

17. Nucleic acid with bacterial promoter function comprising a polynucleotide having at least 80 % nucleotide identity with the sequence SEQ ID No.3, or a polynucleotide with a complementary sequence.

18. Nucleic acid comprising from the 5' end to the 3' end a first nucleic acid according to Claim 14 and a second nucleic acid comprising all or part of a polynucleotide coding for a 1,3-propanediol dehydrogenase having at least 90 % nucleotide identity with the polynucleotide of sequence SEQ ID No.4.

19. Nucleic acid according to Claim 18, **characterized in that** it is a polynucleotide having at least 50 % nucleotide identity with the nucleotide sequence SEQ ID No.5.

20. Nucleic acid according to Claim 18, **characterized in that** it comprises in addition a third nucleic acid coding for a glycerol-3-phosphate dehydrogenase and a fourth nucleic acid coding for a glycerol-3-phosphatase.

21. Recombinant cloning and/or expression vector comprising a nucleic acid according to one of Claims 14 to 20.

22. Recombinant vector according to Claim 21, **characterized in that** it is the plasmid pSPD5 contained in the *Escherichia coli* strain filed at the National Collection of Cultures of Micro-organisms (NCCM) on 24 June 1999 under the access No.I-2243.

23. Recombinant host cell comprising a nucleic acid according to one of Claims 14 to 20 or a recombinant vector according to one of Claims 21 and 22.

24. Recombinant host cell according to Claim 23, **characterized in that** it is the *Escherichia coli* strain filed at the National Collection of Cultures of Micro-organisms (NCCM) on 24 June 1999 under the access No.I-2243.

25. Polypeptide encoded in a nucleic acid according to one of Claims 13 or 14, comprising at least 20 consecutive amino/acids of an amino acid sequence having at least 50 % amino acid identity with the sequence SEQ ID No.6 or SEQ ID No.7.

26. Dimeric protein composed of a first polypeptide having at least 50 % amino acid identity with the polypeptide of sequence SEQ ID No.6 and of a second polypeptide having at least 50 % amino acid identity with the polypeptide of sequence SEQ ID No.7.

27. Process for the production of a polypeptide according to one of Claims 25 and 26, **characterized in that** it comprises the following steps :
a) preparation of a recombinant expression vector according to one of Claims 21 and 22 ;
b) introduction of the recombinant expression vector of step a) into a suitable host cell ;
c) culture of the recombinant host cell of step b) into a suitable culture medium ;
d) recovery of the recombinant polypeptide produced from the culture supernatant or from the cell lysate ;
e) if necessary, purification of the polypeptide recovered.

28. Antibody specifically directed against a polypeptide selected from the polypeptides of the amino acid sequences SEQ ID No.6 or SEQ ID No.7.

## Patentansprüche

1. Verfahren zur Herstellung von 1,3-Propandiol ausgehend von einer kohlenstoffhaltigen Substanz, wobei das Verfahren wenigstens eine Stufe der Kultur eines rekombinanten Mikroorganismus umfasst, in den wenigstens eine Nucleinsäure eingeführt ist, die für die zwei Untereinheiten einer Glycerindehydratase codiert, deren katalytische Aktivität vom Vorliegen von Co-Enzym B12 oder einem seiner Vorläufer unabhängig ist, wobei dieses dimere Protein aus einem ersten Polypeptid, das wenigstens 50% Identität der Aminosäuren mit dem Polypeptid der Sequenz SEQ ID NO: 6 hat, und einem zweiten Polypeptid, das wenigstens 50% Identität der Aminosäuren mit dem Polypeptid der Sequenz SEQ ID NO: 7 hat, besteht.

2. Verfahren nach Anspruch 1, **dadurch gekennzeichnet, dass** die Glycerindehydratase, deren katalytische Aktivität vom Vorliegen von Co-Enzym B12 oder einem seiner Vorläufer unabhängig ist, aus *Clostridium butyricum* stammt.

3. Verfahren nach einem der Ansprüche 1 und 2, **dadurch gekennzeichnet, dass** der rekombinante Mikroorganismus außerdem eine Nucleinsäure umfasst, die für eine 1,3-Propandioldehydrogenase und vorzugsweise eine 1,3-Propandioldehydrogenase von *Clostridium butyricum* VPI 1718 codiert.

4. Verfahren nach Anspruch 3, **dadurch gekennzeichnet, dass** die 1,3-Propandioldehydrogenase ein Polypeptid ist, das wenigstens 90% Identität der Aminosäure mit dem Polypeptid der Sequenz SEQ ID NO. 8 hat.

5. Verfahren nach einem der Ansprüche 1 bis 4, **dadurch gekennzeichnet, dass** die Stufe der Kultur des rekombinanten Mikroorganismus in Abwesenheit des Co-Enzyms B12 oder einer seiner Vorläufer durchgeführt wird.

6. Verfahren nach einem der Ansprüche 1 bis 5, **dadurch gekennzeichnet, dass** die kohlenstoffhaltige Substanz unter den Osiden und den Polyolen ausgewählt wird.

7. Verfahren nach Anspruch 6, **dadurch gekennzeichnet, dass** das Osid Glucose ist und das Polyol Glycerin ist.

8. Verfahren nach einem der Ansprüche 1 bis 7, **dadurch gekennzeichnet, dass** der Mikroorganismus unter den Mikroorganismen ausgewählt wird, die natürlicher Weise das Co-Enzym B12 oder einen seiner Vorläufer nicht produzieren.

9. Verfahren nach Anspruch 8, **dadurch gekennzeichnet, dass** es sich um ein Bakterium, eine Hefe oder einen Pilz handelt.

10. Verfahren nach Anspruch 9, **dadurch gekennzeichnet, dass** es sich um ein Bakterium handelt, dass zur Gattung *Clostridium, Escherichia, Bacillus, Lactobacillus* oder *Lactococcus* gehört.

11. Verfahren nach Anspruch 9, **dadurch gekennzeichnet, dass** es sich um die Hefe *Saccharomyces cerevisiae* handelt.

12. Verfahren nach einem der Ansprüche 1 bis 11, **dadurch gekennzeichnet, dass** der rekombinante Mikroorganismus auch Nucleinsäuren umfasst, die für eine Glycerin-3-phosphatdehydrogenase bzw. eine Glycerin-3-phosphatase codieren.

13. Nucleinsäure, umfassend wenigstes 30 fortlaufende Nucleotide eines Polynucleotids, das für wenigstens eine Untereinheit einer Glycerindehydratase codiert, deren katalytische Aktivität vom Vorliegen von Co-Enzym B12 oder einem seiner Vorläufer unabhängig ist, wobei die Nucleinsäure ein Polynucleotid ganz oder teilweise umfasst, das wenigstens 50% Identität der Nucleotide mit dem Polynucleotid der Sequenz SEQ ID NO. 1 oder SEQ ID NO: 2 oder einem Polynucleotid mit komplementärer Sequenz hat.

14. Nucleinsäure nach Anspruch 13, **dadurch gekennzeichnet, dass** sie ein erstes Polynucleotid, das wenigstens 50% Identität der Nucleotide mit dem Polynucleotid der Sequenz SEQ ID NO: 1 hat, und ein zweites Polynucleotid, das wenigstens 50% Identität der Nucleotide mit dem Polynucleotid der Sequenz SEQ ID NO: 2 hat, umfasst.

15. Nucleinsäure nach Anspruch 14, **dadurch gekennzeichnet, dass** sie außerdem eine Sequenz mit Funktion als Promotor der Transkription umfasst, die in einer Wirtszelle funktionell ist, in der die Expression des Polynucleotids erforscht wird.

16. Nucleinsäure nach Anspruch 15, **dadurch gekennzeichnet, dass** die Promotorsequenz die Sequenz SEQ ID NO: 3 oder eine Sequenz, die wenigstens 80% Identität der Nucleotide mit dieser letztgenannten hat, ist.

17. Nucleinsäure mit der Funktion eines bakteriellen Promotors, die ein Polynucleotid, das wenigstens 80% Identität der Nucleotide mit der Sequenz SEQ ID NO: 3 hat, oder ein Polynucleotid komplementärer Sequenz umfasst.

18. Nucleinsäure, umfassend vom 5'-Ende zum 3'-Ende eine erste Nucleinsäure nach Anspruch 14 und eine zweite Nucleinsäure, die ein Polynucleotid, das für eine 1,3-Propandioldehydrogenase codiert, das wenigstens 90% Identität der Nucleotide mit dem Polynucleotid der Sequenz SEQ ID NO: 4 hat, codiert, ganz oder teilweise umfasst.

19. Nucleinsäure nach Anspruch 18, **dadurch gekennzeichnet, dass** es sich um ein Polynucleotid handelt, das wenigstens 50% Identität der Nucleotide mit der Nucleotidsequenz SEQ ID NO: 5 hat.

20. Nucleinsäure nach Anspruch 18, **dadurch gekennzeichnet, dass** sie außerdem eine dritte Nucleinsäure, die für eine Glycerin-3-phosphatdehydrogenase codiert, und eine vierte Nucleinsäure, die für eine Glycerin-3-phosphatase codiert, umfasst.

21. Rekombinanter Klonierungs- und/oder Expressionsvektor, der eine Nucleinsäure nach einem der Ansprüche 14 bis 20 umfasst.

22. Rekombinanter Vektor nach Anspruch 21, **dadurch gekennzeichnet, dass** es sich um das Plasmid pSPD5 handelt, das in dem *Escherichiacoli-Stamm* enthalten ist, der bei der Collection Nationale de Cultures de Microorganismes (CNCM) am 24. Juni 1999 unter der Eingangsnummer 1-2243 hinterlegt wurde.

23. Rekombinante Wirtszelle, die eine Nucleinsäure nach einem der Ansprüche 14 bis 20 oder einen rekombinanten Vektor nach einem der Ansprüche 21 und 22 umfasst.

24. Rekombinante Wirtszelle nach Anspruch 23, **dadurch gekennzeichnet, dass** es sich um den *Escherichiacoli-Stamm* handelt, der am 24. Juni 1999 unter der Eingangsnummer 1-2243 bei der Collection Nationale de Cultures de Micro-organismes (CNCM) hinterlegt wurde.

25. Polypeptid, das durch eine Nucleinsäure nach einem der Ansprüche 13 und 14 codiert wird, das wenigstens 20 aufeinanderfolgende Aminosäuren einer Sequenz von Aminosäuren umfasst, die wenigstens 50% Identität der Aminosäuren mit der Sequenz SEQ ID NO: 6 oder SEQ ID NO: 7 hat.

26. Dimeres Protein, bestehend aus einem ersten Polypeptid, das wenigstens 50% Identität der Aminosäuren mit dem Polypeptid der Sequenz SEQ ID NO: 6 hat, und einem zweitem Polypeptid, das wenigstens 50% Identität der Aminosäuren mit dem Polypeptid der Sequenz SEQ ID NO: 7 hat.

27. Verfahren zur Herstellung eines Polypeptids nach einem der Ansprüche 25 und 26, **dadurch gekennzeichnet, dass** es die folgenden Stufen umfasst:
a) Herstellung eines rekombinanten Expressionsvektors nach einem der Ansprüche 21 und 22;
b) Einführung des rekombinanten Expressionsvektors der Stufe a) in eine geeignete Wirtszelle;
c) Kultur der rekombinanten Wirtszelle der Stufe b) in einem geeigneten Kulturmedium;
d) Gewinnung des produzierten rekombinanten Polypeptids im Kulturüberstand oder Zelllysat;
e) gegebenenfalls Reinigung des Polypeptids.

28. Antikörper, der spezifisch gegen ein Polypeptid gerichtet ist, das unter den Polypeptiden der Aminosäuresequenz SEQ ID NO: 6 und SEQ ID NO: 7 ausgewählt ist.
